# EUROPÄISCHE PATENTSCHRIFT

(11) **EP 0 915 981 B1**
(45) Veröffentlichungstag und Bekanntmachung des Hinweises auf die Patenterteilung: **29.11.2006**
(21) Anmeldenummer: 97927087.3
(22) Anmeldetag: 30.05.1997
(51) Int. Cl.: C12N 15/52, C12N 9/00, C07K 14/36, C12Q 1/68, C12N 15/63, C12N 1/21, C12P 19/00

(54) **ISOLIERUNG DER BIOSYNTHESEGENE FÜR PSEUDO-OLIGOSACCHARIDE AUS STREPTOMYCES GLAUCESCENS GLA.O UND IHRE VERWENDUNG**
ISOLATION OF THE BIOSYNTHESIS GENES FOR PSEUDO-OLIGOSACCHARIDES FROM STREPTOMYCES GLAUCESCENS GLA.O AND THEIR USE
PROCEDE POUR ISOLER DES GENES DE BIOSYNTHESE POUR PSEUDO-OLIGOSACCHARIDES ISSUS DE STREPTOMYCES GLAUCESCENS GLA.O ET LEUR UTILISATION

(30) Priorität: 07.06.1996 DE 19622783
(43) Veröffentlichungstag der Anmeldung: 19.05.1999
(73) Patentinhaber: Sanofi-Aventis Deutschland GmbH, 65929 Frankfurt am Main (DE)
(72) Erfinder: DECKER, Heinrich, D-65817 Bremtal (DE)
(86) Internationale Anmeldenummer: PCT/EP1997/002826
(87) Internationale Veröffentlichungsnummer: WO 1997/047748

(56) Entgegenhaltungen:
- EP-A- 0 730 029
- DE-A- 2 209 834
- FEMS MICROBIOLOGY LETTERS, Bd. 90, Nr. 2, 1.Januar 1992, Seiten 185-190, XP002040339 MICHAEL STOCKMANN ET AL.: "Gene probes for the detection of 6-deoxyhexose metabolism in secondary metabolite-producing Streptomyces" in der Anmeldung erwähnt

### SEQUENZPROTOKOLL

(1) ALLGEMEINE INFORMATION:
   (i) ANMELDER:
      (A) NAME: Hoechst Aktiengesellschaft
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-3005
      (H) TELEFAX: 069-35-7175
      (I) TELEX: -
   (ii) ANMELDETITEL: Isolierung der Biosynthesegene fuer Pseudo-Oligosaccharide aus Streptomyces glaucescens GLA.O und ihre Verwendung
   (iii) ANZAHL DER SEQUENZEN: 13
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1:
   (i) SEQUENZ CHARAKTERISTIKA:
      (A) LÄNGE: 22 Basenpaare
      (B) ART: Nukleinsäure
      (C) STRANGFORM: Einzel
      (D) TOPOLOGIE: linear
   (ii) ART DES MOLEKÜLS: DNS
   (ix) MERKMALE:
      (A) NAME/SCHLÜSSEL: exon
      (B) LAGE: 1..22
   (xi) SEQUENZBESCHREIBUNG: SEQ ID NO: 1:
      CSGGSGSSGC SGGSTTCATS GG 22
(2) INFORMATION ZU SEQ ID NO: 2:
   (i) SEQUENZ CHARAKTERISTIKA:
      (A) LÄNGE: 24 Basenpaare
      (B) ART: Nukleinsäure
      (C) STRANGFORM: Einzel
      (D) TOPOLOGIE: linear
   (ii) ART DES MOLEKÜLS: DNS
   (ix) MERKMALE:
      (A) NAME/SCHLÜSSEL: exon
      (B) LAGE: 1..24
   (xi) SEQUENZBESCHREIBUNG: SEQ ID NO: 2:
      GGGWVCTGGY VSGGSCCGTA GTTG 24
(2) INFORMATION ZU SEQ ID NO: 3:
   (i) SEQUENZ CHARAKTERISTIKA:
      (A) LÄNGE: 546 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..546
   (xi) SEQUENZBESCHREIBUNG: SEQ ID NO: 3:
   CCCGGGCGGG GCGGGGTTCA TCGGCTCCGC CTACGTCCGC CGGCTCCTGT CGCCCGGGGC    60
   CCCCGGCGGC GTCGCGGTGA CCGTCCTCGA CAAACTCACC TACGCCGGCA GCCTCGCCCG   120
   CCTGCACGCG GTGCGTGACC ATCCCGGCCT CACCTTCGTC CAGGGCGACG TGTGCGACAC   180
   CGCGCTCGTC GACACGCTGG CCGCGCGGCA CGACGACATC GTGCACTTCG CGGCCGAGTC   240
   GCACGTCGAC CGCTCCATCA CCGACAGCGG TGCCTTCACC CGCACCAACG TGCTGGGCAC   300
   CCAGGTCCTG CTCGACGCCG CGCTCCGCCA CGGTGTGCGC ACCCTCGTGC ACGTCTCCAC   360
   CGACGAGGTG TACGGCTCCC TCCCGCACGG GGCCGCCGCG GAGAGCGACC CCCTGCTCCC   420
   GACCTCGCCG TACGCGGCGT CGAAGGCGGC CTCGGACCTC ATGGCGCTCG CCCACCACCG   480
   CACCCACGGC CTGGACGTCC GGGTGACCCG CTGTTCGAAC AACTACGGCC CGCACCAGTT   540
   CCCGGG                                                              546
(2) INFORMATION ZU SEQ ID NO: 4:
   (i) SEQUENZ CHARAKTERISTIKA:
      (A) LÄNGE: 541 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..541
   (xi) SEQUENZBESCHREIBUNG: SEQ ID NO: 4:
   CCCCGGGTGC TGGTAGGGGC CGTAGTTGTT GGAGCAGCGG GTGATGCGCA CGTCCAGGCC    60
   GTGGCTGACG TGCATGGCCA GCGCGAGCAG GTCGCCCGAC GCCTTGGAGG TGGCATAGGG   120
   GCTGTTGGGG CGCAGCGGCT CGTCCTCCGT CCACGACCCC GTCTCCAGCG AGCCGTAGAC   180
   CTCGTCGGTG GACACCTGCA CGAAGGGGGC CACGCCGTGC CGCAGGGCCG CGTCGAGGAG   240
   TGTCTGCGTG CCGCCGGCGT TGGTCCGCAC GAACGCGGCG GCATCGAGCA GCGAGCGGTC   300
   CACGTGCGAC TCGGCGGCGA GGTGCACGAC CTGGTCCTGG CCGGCCATGA CCCGGTCGAC     360
   CAGGTCCGCG TCGCAGATGT CGCCGTGGAC GAAGCGCAGC CGGGGGTGGT CGCGGACCGG     420
   GTCGAGGTTG GCGAGGTTGC CGGCGTAGCT CAGGGCGTCG AGCACGGTGA CGACGGCGTC     480
   GGGCGGCCCG TCCGGACCGA GGAGGGTGCG GACGTAGTGC GAGCCCATGA ACCCCGCCGC     540
   C                                                                    541
(2) INFORMATION ZU SEQ ID NO: 5:
   (i) SEQUENZ CHARAKTERISTIKA:
      (A) LÄNGE: 180 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: PCRstrE.Pep
      (B) LAGE: 1..180
   (xi) SEQUENZBESCHREIBUNG: SEQ ID NO: 5:
   Ala Ala Gly Phe Met Gly Ser His Tyr Val Arg Thr Leu Leu Gly Pro
   1               5                   10                  15
   Asp Gly Pro Pro Asp Ala Val Val Thr Val Leu Asp Ala Leu Ser Tyr
               20                  25                  30
   Ala Gly Asn Leu Ala Asn Leu Asp Pro Val Arg Asp His Pro Arg Leu
           35                  40                  45
   Arg Phe Val His Gly Asp Ile Cys Asp Ala Asp Leu Val Asp Arg Val
       50                  55                  60
   Met Ala Gly Gln Asp Gln Val Val His Leu Ala Ala Glu Ser His Val
   65                  70                  75                  80
   Asp Arg Ser Leu Leu Asp Ala Ala Ala Phe Val Arg Thr Asn Ala Gly
                   85                  90                  95
   Gly Thr Gln Thr Leu Leu Asp Ala Ala Leu Arg His Gly Val Ala Pro
               100                 105                 110
   Phe Val Gln Val Ser Thr Asp Glu Val Tyr Gly Ser Leu Glu Thr Gly
           115                 120                 125
   Ser Trp Thr Glu Asp Glu Pro Leu Arg Pro Asn Ser Pro Tyr Ala Thr
       130                 135                 140
   Ser Lys Ala Ser Gly Asp Leu Leu Ala Leu Ala Met His Val Ser His
   145                 150                 155                 160
   Gly Leu Asp Val Arg Ile Thr Arg Cys Ser Asn Asn Tyr Gly Pro Tyr
                   165                 170                 175
   Gln His Pro Gly
               180
(2) INFORMATION ZU SEQ ID NO: 6:
   (i) SEQUENZ CHARAKTERISTIKA:
      (A) LÄNGE: 181 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: PCR acbD.Pep
      (B) LAGE: 1..181
   (xi) SEQUENZBESCHREIBUNG: SEQ ID NO: 6:
   Pro Gly Gly Ala Gly Phe Ile Gly Ser Ala Tyr Val Arg Arg Leu Leu
   1               5                   10                  15
   Ser Pro Gly Ala Pro Gly Gly Val Ala Val Thr Val Leu Asp Lys Leu
               20                  25                  30
   Thr Tyr Ala Gly Ser Leu Ala Arg Leu His Ala Val Arg Asp His Pro
           35                  40                  45
   Gly Leu Thr Phe Val Gln Gly Asp Val Cys Asp Thr Ala Leu Val Asp
       50                  55                  60
   Thr Leu Ala Ala Arg His Asp Asp Ile Val His Phe Ala Ala Glu Ser
   65                  70                  75                  80
   His Val Asp Arg Ser Ile Thr Asp Ser Gly Ala Phe Thr Arg Thr Asn
                   85                  90                  95
   Val Leu Gly Thr Gln Val Leu Leu Asp Ala Ala Leu Arg His Gly Val
               100                 105                 110
   Arg Thr Leu Val His Val Ser Thr Asp Glu Val Tyr Gly Ser Leu Pro
           115                 120                 125
   His Gly Ala Ala Ala Glu Ser Asp Pro Leu Leu Pro Thr Ser Pro Tyr
       130                 135                 140
   Ala Ala Ser Lys Ala Ala Ser Asp Leu Met Ala Leu Ala His His Arg
   145                 150                 155                 160
   Thr His Gly Leu Asp Val Arg Val Thr Arg Cys Ser Asn Asn Tyr Gly
                   165                 170                 175
   Pro His Gln Phe Pro
               180
(2) INFORMATION ZU SEQ ID NO: 7:
   (i) SEQUENZ CHARAKTERISTIKA:
      (A) LÄNGE: 6854 Basenpaare
      (B) ART: Nukleinsäure
      (C) STRANGFORM: Doppel
      (D) TOPOLOGIE: linear
   (ii) ART DES MOLEKÜLS: DNS (genomisch)

   (ix) MERKMALE:
      (A) NAME/SCHLÜSSEL: Biosynthesegencluster "Acarbose"
      (B) LAGE: 1..6854
   (xi) SEQUENZBESCHREIBUNG: SEQ ID NO: 7:
   CTGCAGGGTT CCCTGGTGCA CGACCCGCCC CTGGTCGACG ACCAGGGCGC TGTCGCAGAT 60
   CGCGGCGATG TCGGCGATGT CGTGGCTGGT GAGCACCACG GTGGTGCCCA GTTCCCGGTG 120
   GGCGCGGTTG ACCAGCCGGC GCACCGCGTC CTTCAGCACC ATGTCGAGGC CGATCGTGGG 180
   CTCGTCCCAG AACAGCACGG CCGGGTCGTG CAGCAGGCTC GCCGCGATCT CGGCGCGCAT 240
   GCGCTGTCCG AGGCTGAGCT GCCGCACGGG GGTGGACCCC AGCGCGTCGA TGTCGAGGAG 300
   GTCCCGGAAC AGGGCGAGGT TGCGCCGGTA GACCGGTCCG GGGATGTCGT AGATGCGGCG 360
   CAGGATGCGG AAGGAGTCGG GTACCGACAG GTCCCACCAG AGCTGGCTGC GCTGGCCGAA 420
   GACGACGCCG ATCGTGCGGG CGTTGCGCTG CCGGTGCCGG TAGGGCTCCA GCCCGGCGAC 480
   CGTGCAGCGG CCGGAGGTGG GGGTCATGAT GCCGGTCAGC ATCTTGATCG TGGTCGACTT 540
   GCCGGCTCCG TTGGCGCCGA TGTAGGCGGT CTTCGTGCCG GCCGGTATCT CGAAGGAGAC 600
   GTCGTCGACG GCGCGCACGA CGCGGTACCG GCGGGTCAGG AGGGTGGAGA GGCTGCCGAG 660
   CAGGCCGGGC TCGCGTTCGG CCAGCCGGAA CTCCTTGACG AGGTGTTCGG CCACGATCAC 720
   GCGATCACCC GCTCGACGGC CGTCTCCAGC AGGCGCAGGC CCTCGTCGAG CAGCGCCTCG 780
   TCGAGGGTGA ACGGCGGTGC CAGCCGCAGG ATGTGGCCGC CCAGGGAGGT GCGCAGCCCC 840
   AGGTCGAGGG CGGTGGTGTA GACGGCCCGG GCGGTCTCGG GGGCGGGTGC CCGGCCGACG 900
   GCGTCGGTGA CGAACTCCAG GCCCCACAGC AGTCCGAGGC CGCGTACCTG GCCGAGCTGG 960
   GGGAAGCGGG ACTCCAGGGC GCGCAGCCGC TCCTGGATGA GCTCGCCGAG GACGCGCACG 1020
   CGGTCGATCA GCCGGTCGCG CTCGACGACC TCCAGCGTGG CGCGGGCGGC GGCGATCCCC 1080
   AGTGGGTTGC TCGCGTACGT CGAGGCGTAC GCCCCGGGGT GGCCGCCTCC GGCCTGCGCA 1140
   GCTTCCGCGC GTCCGGCCAG CACGGCGAAG GGGAATCCGC TCGCGGTGCC CTTGGACAGC 1200
   ATCGCCAGGT CCGGCTCGAT GCCGAACAGT TCGCTGGCGA GGAAGGCGCC GGTGCGCCCG 1260
   CCGCCGGTGA GGACCTCGTC GGCGACGAGC AGCACGCCGC CGTCCCGGCA GGCGCCGGCG 1320
   ATCCGCTCCC AGTAGCCGGG GGGCGGCACG ATGACGCCTG CCGCGCCGAG GACGGGTTCG 1380
   AAGACCAGGG CCGAGACGTT GGGCTTCTCC GCGATGTGCC GGCGCACGAG GGTCGCGCAC 1440
   CGCACGTCGC ACGAGGGGTA CTCCAGGCCC AGGGGACAGC GGTAGCCAGT AGGGGCTGTA 1500
   GCCAGCACGC TGTTGCCGCT GAAGGCCTGG TGGCCGATGT CCCAGTGGAC CAGCATCCGG 1560
   GCGCCCATGG TCTTGCCGTG GAAGCCGTGG CGCAGGGCGC AGATCCGGTT GCGGCCCGGC 1620
   GCGGCGGTCG CCTGGACGAC CCGCAGGGCG GCCTCGACCA CCTCCGCGCC GGTGGAGAAG 1680
   AAGGCGTAGG TGTCGAGCTG TTCGGGCAGC AGCCTGGCGA GCAGTTCCAG CAGGCCGGCG 1740
   CGGTCCGGCG TGGCGCTGTC GTGGACGTTC CACAGGCGGC GGGCCTGGGT GGTGAGTGCC 1800
   TCGACGACCT CCGGGTGCCC GTGGCCCAGT GACTGGGTGA GGGTCCCGGC CGCGAAGTCG 1860
   AGGTACTGGT TGCCGTCCAG GTCGGTCAGA ACGGGACCGC GTCCCTCGGC GAAGACCCGG 1920
   CGTCCGTGGA CGGCTTCCTC GGAGGCGCCC GGCGCCAGGT GGCGGGCCTC CCGTGCCAGG 1980
   TGCTGTGTCT GCCGTAAGCC TGTCATCGCT GCCTCTGCTC GTCGGACCGG CTGACGCGAT 2040
   CGCCGGCGAA CTGCGTTGTG GCGCACCACG GTTGGGGCGG CTCGGCGCTG AGTCAAACAC 2100
   TTGAACACAC ACCGCTGCAA GAGTTTGCGG GTTGTTTCAG AAAGTTGTTG CGAGCGGCCC 2160
   CGGCACTCTG GTTGAGTCGA CGTGCTTACG GCGCCACCAC GCCTCACGTT CGAGGAGGGA 2220
   CCTGTGAGAA CAAGCCCGCA GACCGACCCG CTCCCGCGGA GGCCGAGGTG AAGGCCCTGG 2280
   TCCTGGCAGG TGGAACCGGC AGCAGACTGA GGCCGTTCAC CCACACCGCC GCCAAGCAGC 2340
   TGCTCCCCAT CGCCAACAAG CCCGTGCTCT TCTACGCGCT GGAGTCCCTC GCCGCGGCGG 2400
   GTGTCCGGGA GGCCGGCGTC GTCGTGGGCG CGTACGGCCG GGAGATCCGC GAACTCACCG 2460
   GCGACGGCAC CGCGTTCGGG TTACGCATCA CCTACCTCCA CCAGCCCCGC CCGCTCGGTC 2520
   TCGCGCACGC GGTGCGCATC GCCCGCGGCT TCCTGGGCGA CGACGACTTC CTGCTGTACC 2580
   TGGGGGACAA CTACCTGCCC CAGGGCGTCA CCGACTTCGC CCGCCAATCG GCCGCCGATC 2640
   CCGCGGCGGC CCGGCTGCTG CTCACCCCGG TCGCGGACCC GTCCGCCTTC GGCGTCGCGG 2700
   AGGTCGACGC GGACGGGAAC GTGCTGCGCT TGGAGGAGAA ACCCGACGTC CCGCGCAGCT 2760
   CGCTCGCGCT CATCGGCGTG TACGCCTTCA GCCCGGCCGT CCACGAGGCG GTACGGGCCA 2820
   TCACCCCCTC CGCCCGCGGC GAGCTGGAGA TCACCCACGC CGTGCAGTGG ATGATCGACC 2880
   GGGGCCTGCG CGTACGGGCC GAGACCACCA CCCGGCCCTG GCGCGACACC GGCAGCGCGG 2940
   AGGACATGCT GGAGGTCAAC CGTCACGTCC TGGACGGACT GGAGGGCCGC ATCGAGGGGA 3000
   AGGTCGACGC GCACAGCACG CTGGTCGGCC GGGTCCGGGT GGCCGAAGGC GCGATCGTGC 3060
   GGGGGTCACA CGTGGTGGGC CCGGTGGTGA TCGGCGCGGG TGCCGTCGTC AGCAACTCCA 3120
   GTGTCGGCCC GTACACCTCC ATCGGGGAGG ACTGCCGGGT CGAGGACAGC GCCATCGAGT 3180
   ACTCCGTCCT GCTGCGCGGC GCCCAGGTCG AGGGGGCGTC CCGCATCGAG GCGTCCCTCA 3240
   TCGGCCGCGG CGCCGTCGTC GGCCCGGCCC CCCGTCTCCC GCAGGCTCAC CGACTGGTGA 3300
   TCGGCGACCA CAGCAAGGTG TATCTCACCC CATGACCACG ACCATCCTCG TCACCGGCGG 3360
   AGCGGGCTTC ATTCGCTCCG CCTACGTCCG CCGGCTCCTG TCGCCCGGGG CCCCCGGCGG 3420
   CGTCGCGGTG ACCGTCCTCG ACAAACTCAC CTACGCCGGC AGCCTCGCCC GCCTGCACGC 3480
   GGTGCGTGAC CATCCCGGCC TCACCTTCGT CCAGGGCGAC GTGTGCGACA CCGCGCTCGT 3540
   CGACACGCTG GCCGCGCGGC ACGACGACAT CGTGCACTTC GCGGCCGAGT CGCACGTCGA 3600
   CCGCTCCATC ACCGACAGCG GTGCCTTCAC CCGCACCAAC GTGCTGGGCA CCCAGGTCCT 3660
   GCTCGACGCC GCGCTCCGCC ACGGTGTGCG CACCTTCGTG CACGTCTCCA CCGACGAGGT 3720
   GTACGGCTCC CTCCCGCACG GGGCCGCCGC GGAGAGCGAC CCCCTGCTTC CGACCTCGCC 3780
   GTACGCGGCG TCGAAGGCGG CCTCGGACCT CATGGCGCTC GCCCACCACC GCACCCACGG 3840
   CCTGGACGTC CGGGTGACCC GCTGTTCGAA CAACTTCGGC CCCCACCAGC ATCCCGAGAA 3900
   GCTCATACCG CGCTTCCTGA CCAGCCTCCT GTCCGGCGGC ACCGTTCCCC TCTACGGCGA 3960
   CGGGCGGCAC GTGCGCGACT GGCTGCACGT CGACGACCAC GTCAGGGCCG TCGAACTCGT 4020
   CCGCGTGTCG GGCCGGCCGG GAGAGATCTA CAACATCGGG GGCGGCACCT CGCTGCCCAA 4080
   CCTGGAGCTC ACGCACCGGT TGCTCGCACT GTGCGGCGCG GGCCCGGAGC GCATCGTCCA 4140
   CGTCGAGAAC CGCAAGGGGC ACGACCGGCG CTACGCGGTC GACCACAGCA AGATCACCGC 4200
   GGAACTCGGT TACCGGCCGC GCACCGACTT CGCGACCGCG CTGGCCGACA CCGCGAAGTG 4260
   GTACGAGCGG CACGAGGACT GGTGGCGTCC CCTGCTCGCC GCGACATGAC GTCGGGCCGG 4320
   ACCGCAACCA CCGGCCCCGG CCGGCACACC GCCGCCCGCG GCCGGTGGCC GGCCGGTCAG 4380
   CGTCCGTGAG CCGGGCGCCG GCCGCCCCGC GGGCCGGCGG CGGTGGACCC CCGGACCACC 4440
   AGTTCCGGCA TGAAGACGAA TTCGGTGCGC GGCGGCGGCG TTCCGCTCAT CTCCTCCAGC 4500
   AGTGCGTCCA CGGCGACCTG CCCCATCGCC TTGACGGGCT GTCTGATGGT GGTCAGGGGA 4560
   GGGTCGGTGA AGGCCATGAG CGGCGAGTCG TCGAAGCCGA CCACCGAGAT GTCACCGGGA 4620
   ACCGTGAGAC CCCGCCGGCG CGCGGCCCGC ACGGCGCCGA GGGCCATCAT GTCGCTGGCG 4680
   CACATGACGG CGGTGCAGCC CAGGTCGATC AGCGCGGACG CGGCGGCCTG GCCCCCCTCC 4740
   AGGGAGAACA GCGAGTGCTG CACGAGCTCC TCGGACTCCC GCGCCGACAC TCCCAGGTGC 4800
   TCCCGCACGC CGGCCCGGAA CCCCTCGATC TTCCGCTGCA CCGGCACGAA GCGGGCGGGC 4860
   CCGACGGCGA GGCCGACGCG CTCGTGCCCC AGCTCCGCCA GGTGCGCCAC GGCCAGGCGC 4920
   ATCGCGGCCC GGTCGTCCGG GGAGACGAAG GGTGCCTCGA TCCGGGGCGA GAACCCGTTC 4980
   ACGAGGACGA AGGGCACCTG CCGCTCGTGC AGCCGGCCGT ACCGTCCGGT CTCGGCGGTG 5040
   GTGTCCGCGT GCAGTCCGGA GACGAAGATG ATGCCGGACA CCCCGCGGTC CACGAGCATC 5100
   TCCGTGAGTT CGTCCTCGGT CGAGCCGCCC GGGGTCTGCG TGGCGAGCAC GGGCGTGTAG 5160
   CCCTGACGCG TGAGCGCCTG CCCCATCACC TGGGCCAGTG CGGGGAAGAA GGGGTTGTCC 5220
   AGTTCGGGGG TGACCAGTCC GACCAGCTCG GCGCGGCGCT GTCGCGCCGG CTGCTCGTAG 5280
   CCCAGCGCGT CCAGTGCGGT CAGCACCGAG TCGCGGGTGC CGGTGGCCAC ACCGCGCGCA 5340
   CCGTTCAGCA CCCGGCTGAC CGTGGCCTTG CTGACGCCCG CCCGGGCTGC GATGTCGGCG 5400
   AGCCGCATGG TCATGGCAAC GCACTCTACC TGTCGGGGCG TCAGGGCGTG CCCACCGCGC 5460
   GCGGAACCGG CGGACTGCGG GGCACGGCCC GTCCGCCGCC CACGGACCAC GCGCCCGAAA 5520
   CGATGGCTGA AAATGCTTGC AGCAAATTGC CGCAACGTCT TTCGGCGGCT TTTCGATCCT 5580
   GTTACGTTCC TGGCAACCCC GGCGCCGCGC AGAAGCGGTT GGCGTGAGGC GTCCAGACCT 5640
   CCGCCCGATT CCGGGATCAC TCAGGGGAGT TCACAATGCG GCGTGGCATT GCGGCCACCG 5700
   CGCTGTTCGC GGCTGTGGCC ATGACGGCAT CGGCGTGTGG CGGGGGCGAC AACGGCGGAA 5760
   GCGGTACCGA CGCGGGCGGC ACGGAGCTGT CGGGGACCGT CACCTTCTGG GACACGTCCA 5820
   ACGAAGCCGA GAAGGCGACG TACCAGGCCC TCGCGGAGGG CTTCGAGAAG GAGCACCCGA 5880
   AGGTCGACGT CAAGTACGTC AACGTCCCGT TCGGCGAGGC GAACGCCAAG TTCAAGAACG  5940
   CCGCGGGCGG CAACTCCGGT GCCCCGGACG TGATGCGTAC GGAGGTCGCC TGGGTCGCGG  6000
   ACTTCGCCAG CATCGGCTAC CTCGCCCCGC TCGACGGCAC GCCCGCCCTC GACGACGGGT  6060
   CGGACCACCT TCCCCAGGGC GGCAGCACCA GGTACGAGGG GAAGACCTAC GCGGTCCCGC  6120
   AGGTGATCGA CACCCTGGCG CTCTTCTACA ACAAGGAACT GCTGACGAAG GCCGGTGTCG  6180
   AGGTGCCGGG CTCCCTCGCC GAGCTGAAGA CGGCCGCCGC CGAGATCACC GAGAAGACCG  6240
   GCGCGAGCGG CCTCTACTGC GGGGCGACGA CCCGTACTTG GTTCCTGCCC TACCTCTACG  6300
   GGGAGGGCGG CGACCTGGTC GACGAGAAGA ACAAGACCGT CACGGTCGAC GACGAAGCCG  6360
   GTGTGCGCGC CTACCGCGTC ATCAAGGACC TCGTGGACAG CAAGGCGGCC ATCACCGACG  6420
   CGTCCGACGG CTGGAACAAC ATGCAGAACG CCTTCAAGTC GGGCAAGGTC GCCATGATGG  6480
   TCAACGGCCC CTGGGCCATC GAGGACGTCA AGGCGGGAGC CCGCTTCAAG GACGCCGGCA  6540
   ACCTGGGGGT CGCCCCCGTC CCGGCCGGCA GTGCCGGACA GGGCTCTCCC CAGGGCGGGT  6600
   GGAACCTCTC GGTGTACGCG GGCTCGAAGA ACCTCGACGC CTCCTACGCC TTCGTGAAGT  6660
   ACATGAGCTC CGCCAAGGTG CAGCAGCAGA CCACCGAGAA GCTGAGCCTG CTGCCCACCC  6720
   GCACGTCCGT CTACGAGGTC CCGTCCGTCG CGGACAACGA GATGGTGAAG TTCTTCAAGC  6780
   CGGCCGTCGA CAAGGCCGTC GAACGGCCGT GGATCGCCGA GGGCAATGCC CTCTTCGAGC  6840
   CGATCCGGCT GCAG                                                    6854
(2) INFORMATION ZU SEQ ID NO: 8:
   (i) SEQUENZ CHARAKTERISTIKA:
      (A) LÄNGE: 240 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: acbA
      (B) LAGE: 1..240
(2) INFORMATION ZU SEQ ID NO: 9:
   (i) SEQUENZ CHARAKTERISTIKA:
      (A) LÄNGE: 429 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: acbB
      (B) LAGE: 1..429
   (xi) SEQUENZBESCHREIBUNG: SEQ ID NO: 9:
   Met Thr Gly Leu Arg Gln Thr Gln His Leu Ala Arg Glu Ala Arg His
   1               5                   10                  15
   Leu Ala Pro Gly Ala Ser Glu Glu Ala Val His Gly Arg Arg Val Phe
               20                  25                  30
   Ala Glu Gly Arg Gly Pro Val Leu Thr Asp Leu Asp Gly Asn Gln Tyr
           35                  40                  45
   Leu Asp Phe Ala Ala Gly Thr Leu Thr Gln Ser Leu Gly His Gly His
       50                  55                  60
   Pro Glu Val Val Glu Ala Leu Thr Thr Gln Ala Arg Arg Leu Trp Asn
   65                  70                  75                  80
   Val His Asp Ser Ala Thr Pro Asp Arg Ala Gly Leu Leu Glu Leu Leu
                   85                  90                  95
   Ala Arg Leu Leu Pro Glu Gln Leu Asp Thr Tyr Ala Phe Phe Ser Thr
   100 105 110
   Gly Ala Glu Val Val Glu Ala Ala Leu Arg Val Val Gln Ala Thr Ala
   115 120 125
   Ala Pro Gly Arg Asn Arg Ile Cys Ala Leu Arg His Gly Phe His Gly
   130 135 140
   Lys Thr Met Gly Ala Arg Met Leu Val His Trp Asp Ile Gly His Gln
   145 150 155 160
   Ala Phe Ser Gly Asn Ser Val Leu Ala Thr Ala Pro Thr Gly Tyr Arg
   165 170 175
   Cys Pro Leu Gly Leu Glu Tyr Pro Ser Cys Asp Val Arg Cys Ala Thr
   180 185 190
   Leu Val Arg Arg His Ile Ala Glu Lys Pro Asn Val Ser Ala Leu Val
   195 200 205
   Phe Glu Pro Val Leu Gly Ala Ala Gly Val Ile Val Pro Pro Pro Gly
   210 215 220
   Tyr Trp Glu Arg Ile Ala Gly Ala Cys Arg Asp Gly Gly Val Leu Leu
   225 230 235 240
   Val Ala Asp Glu Val Leu Thr Gly Gly Gly Arg Thr Gly Ala Phe Leu
   245 250 255
   Ala Ser Glu Leu Phe Gly Ile Glu Pro Asp Leu Ala Met Leu Ser Lys
   260 265 270
   Gly Thr Ala Ser Gly Phe Pro Phe Ala Val Leu Ala Gly Arg Ala Glu
   275 280 285
   Ala Ala Gln Ala Gly Gly Gly His Pro Gly Ala Tyr Ala Ser Thr Tyr
   290 295 300
   Ala Ser Asn Pro Leu Gly Ile Ala Ala Ala Arg Ala Thr Leu Glu Val
   305 310 315 320
   Val Glu Arg Asp Arg Leu Ile Asp Arg Val Arg Val Leu Gly Glu Leu
   325 330 335
   Ile Gln Glu Arg Leu Arg Ala Leu Glu Ser Arg Phe Pro Gln Leu Gly
   340 345 350
   Gln Val Arg Gly Leu Gly Leu Leu Trp Gly Leu Glu Phe Val Thr Asp
   355 360 365
   Ala Val Gly Arg Ala Pro Ala Pro Glu Thr Ala Arg Ala Val Tyr Thr
   370 375 380
   Thr Ala Leu Asp Leu Gly Leu Arg Thr Ser Leu Gly Gly His Ile Leu
   385 390 395 400
   Arg Leu Ala Pro Pro Phe Thr Leu Asp Glu Ala Leu Leu Asp Glu Gly
   405 410 415
   Leu Arg Leu Leu Glu Thr Ala Val Glu Arg Val Ile Ala
   420 425
(2) INFORMATION ZU SEQ ID NO: 10:
   (i) SEQUENZ CHARAKTERISTIKA:
      (A) LÄNGE: 355 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: acbC
      (B) LAGE: 1..355
   (xi) SEQUENZBESCHREIBUNG: SEQ ID NO: 10:
   Val Lys Ala Leu Val Leu Ala Gly Gly Thr Gly Ser Arg Leu Arg Pro
   1               5                   10                  15
   Phe Thr His Thr Ala Ala Lys Gln Leu Leu Pro Ile Ala Asn Lys Pro
               20                  25                  30
   Val Leu Phe Tyr Ala Leu Glu Ser Leu Ala Ala Ala Gly Val Arg Glu
           35                  40                  45
   Ala Gly Val Val Val Gly Ala Tyr Gly Arg Glu Ile Arg Glu Leu Thr
       50                  55                  60
   Gly Asp Gly Thr Ala Phe Gly Leu Arg Ile Thr Tyr Leu His Gln Pro
   65                  70                  75                  80
   Arg Pro Leu Gly Leu Ala His Ala Val Arg Ile Ala Arg Gly Phe Leu
                   85                  90                  95
   Gly Asp Asp Asp Phe Leu Leu Tyr Leu Gly Asp Asn Tyr Leu Pro Gln
               100                 105                 110
   Gly Val Thr Asp Phe Ala Arg Gln Ser Ala Ala Asp Pro Ala Ala Ala
           115                 120                 125
   Arg Leu Leu Leu Thr Pro Val Ala Asp Pro Ser Ala Phe Gly Val Ala
       130                 135                 140
   Glu Val Asp Ala Asp Gly Asn Val Leu Arg Leu Glu Glu Lys Pro Asp
   145                 150                 155                 160
   Val Pro Arg Ser Ser Leu Ala Leu Ile Gly Val Tyr Ala Phe Ser Pro
                   165                 170                 175
   Ala Val His Glu Ala Val Arg Ala Ile Thr Pro Ser Ala Arg Gly Glu
               180                 185                 190
   Leu Glu Ile Thr His Ala Val Gln Trp Met Ile Asp Arg Gly Leu Arg
           195                 200                 205
   Val Arg Ala Glu Thr Thr Thr Arg Pro Trp Arg Asp Thr Gly Ser Ala
       210                 215                 220
   Glu Asp Met Leu Glu Val Asn Arg His Val Leu Asp Gly Leu Glu Gly
   225                 230                 235                 240
   Arg Ile Glu Gly Lys Val Asp Ala His Ser Thr Leu Val Gly Arg Val
                   245                 250                 255
   Arg Val Ala Glu Gly Ala Ile Val Arg Gly Ser His Val Val Gly Pro
   260 265 270
   Val Val Ile Gly Ala Gly Ala Val Val Ser Asn Ser Ser Val Gly Pro
   275 280 285
   Tyr Thr Ser Ile Gly Glu Asp Cys Arg Val Glu Asp Ser Ala Ile Glu
   290 295 300
   Tyr Ser Val Leu Leu Arg Gly Ala Gln Val Glu Gly Ala Ser Arg Ile
   305 310 315 320
   Glu Ala Ser Leu Ile Gly Arg Gly Ala Val Val Gly Pro Ala Pro Arg
   325 330 335
   Leu Pro Gln Ala His Arg Leu Val Ile Gly Asp His Ser Lys Val Tyr
   340 345 350
   Leu Thr Pro
   355
(2) INFORMATION ZU SEQ ID NO: 11:
   (i) SEQUENZ CHARAKTERISTIKA:
      (A) LÄNGE: 325 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: acbD
      (B) LAGE: 1..325
   (xi) SEQUENZBESCHREIBUNG: SEQ ID NO: 11:
   Met Thr Thr Thr Ile Leu Val Thr Gly Gly Ala Gly Phe Ile Arg Ser
   1 5 10 15
   Ala Tyr Val Arg Arg Leu Leu Ser Pro Gly Ala Pro Gly Gly Val Ala
   20 25 30
   Val Thr Val Leu Asp Lys Leu Thr Tyr Ala Gly Ser Leu Ala Arg Leu
   35 40 45
   His Ala Val Arg Asp His Pro Gly Leu Thr Phe Val Gln Gly Asp Val
   50 55 60
   Cys Asp Thr Ala Leu Val Asp Thr Leu Ala Ala Arg His Asp Asp Ile
   65 70 75 80
   Val His Phe Ala Ala Glu Ser His Val Asp Arg Ser Ile Thr Asp Ser
   85 90 95
   Gly Ala Phe Thr Arg Thr Asn Val Leu Gly Thr Gln Val Leu Leu Asp
   100 105 110
   Ala Ala Leu Arg His Gly Val Arg Thr Phe Val His Val Ser Thr Asp
   115 120 125
   Glu Val Tyr Gly Ser Leu Pro His Gly Ala Ala Ala Glu Ser Asp Pro
   130 135 140
   Leu Leu Pro Thr Ser Pro Tyr Ala Ala Ser Lys Ala Ala Ser Asp Leu
   145 150 155 160
   Met Ala Leu Ala His His Arg Thr His Gly Leu Asp Val Arg Val Thr
   165 170 175
   Arg Cys Ser Asn Asn Phe Gly Pro His Gln His Pro Glu Lys Leu Ile
   180 185 190
   Pro Arg Phe Leu Thr Ser Leu Leu Ser Gly Gly Thr Val Pro Leu Tyr
   195 200 205
   Gly Asp Gly Arg His Val Arg Asp Trp Leu His Val Asp Asp His Val
   210 215 220
   Arg Ala Val Glu Leu Val Arg Val Ser Gly Arg Pro Gly Glu Ile Tyr
   225 230 235 240
   Asn Ile Gly Gly Gly Thr Ser Leu Pro Asn Leu Glu Leu Thr His Arg
   245 250 255
   Leu Leu Ala Leu Cys Gly Ala Gly Pro Glu Arg Ile Val His Val Glu
   260 265 270
   Asn Arg Lys Gly His Asp Arg Arg Tyr Ala Val Asp His Ser Lys Ile
   275 280 285
   Thr Ala Glu Leu Gly Tyr Arg Pro Arg Thr Asp Phe Ala Thr Ala Leu
   290 295 300
   Ala Asp Thr Ala Lys Trp Tyr Glu Arg His Glu Asp Trp Trp Arg Pro
   305 310 315 320
   Leu Leu Ala Ala Thr
   325
(2) INFORMATION ZU SEQ ID NO: 12:
   (i) SEQUENZ CHARAKTERISTIKA:
      (A) LÄNGE: 345 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: acbE
      (B) LAGE: 1..345
   (xi) SEQUENZBESCHREIBUNG: SEQ ID NO: 12:
   Met Thr Met Arg Leu Ala Asp Ile Ala Ala Arg Ala Gly Val Ser Lys
   1               5                   10                  15
   Ala Thr Val Ser Arg Val Leu Asn Gly Ala Arg Gly Val Ala Thr Gly
               20                  25                  30
   Thr Arg Asp Ser Val Leu Thr Ala Leu Asp Ala Leu Gly Tyr Glu Gln
           35                  40                  45
   Pro Ala Arg Gln Arg Arg Ala Glu Leu Val Gly Leu Val Thr Pro Glu
       50                  55                  60
   Leu Asp Asn Pro Phe Phe Pro Ala Leu Ala Gln Val Met Gly Gln Ala
   65                  70                  75                  80
   Leu Thr Arg Gln Gly Tyr Thr Pro Val Leu Ala Thr Gln Thr Pro Gly
                   85                  90                  95
   Gly Ser Thr Glu Asp Glu Leu Thr Glu Met Leu Val Asp Arg Gly Val
               100                 105                 110
   Ser Gly Ile Ile Phe Val Ser Gly Leu His Ala Asp Thr Thr Ala Glu
           115                 120                 125
   Thr Gly Arg Tyr Gly Arg Leu His Glu Arg Gln Val Pro Phe Val Leu
       130                 135                 140
   Val Asn Gly Phe Ser Pro Arg Ile Glu Ala Pro Phe Val Ser Pro Asp
   145                 150                 155                 160
   Asp Arg Ala Ala Met Arg Leu Ala Val Ala His Leu Ala Glu Leu Gly
                   165                 170                 175
   His Glu Arg Val Gly Leu Ala Val Gly Pro Ala Arg Phe Val Pro Val
               180                 185                 190
   Gln Arg Lys Ile Glu Gly Phe Arg Ala Gly Val Arg Glu His Leu Gly
           195                 200                 205
   Val Ser Ala Arg Glu Ser Glu Glu Leu Val Gln His Ser Leu Phe Ser
       210                 215                 220
   Leu Glu Gly Gly Gln Ala Ala Ala Ser Ala Leu Ile Asp Leu Gly Cys
   225                 230                 235                 240
   Thr Ala Val Met Cys Ala Ser Asp Met Met Ala Leu Gly Ala Val Arg
                   245                 250                 255
   Ala Ala Arg Arg Arg Gly Leu Thr Val Pro Gly Asp Ile Ser Val Val
               260                 265                 270
   Gly Phe Asp Asp Ser Pro Leu Met Ala Phe Thr Asp Pro Pro Leu Thr
           275                 280                 285
   Thr Ile Arg Gln Pro Val Lys Ala Met Gly Gln Val Ala Val Asp Ala
       290                 295                 300
   Leu Leu Glu Glu Met Ser Gly Thr Pro Pro Pro Arg Thr Glu Phe Val
   305                 310                 315                 320
   Phe Met Pro Glu Leu Val Val Arg Gly Ser Thr Ala Ala Gly Pro Arg
                   325                 330                 335
   Gly Gly Arg Arg Pro Ala His Gly Arg
               340                 345
(2) INFORMATION ZU SEQ ID NO: 13:
   (i) SEQUENZ CHARAKTERISTIKA:
      (A) LÄNGE: 393 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: acbF
      (B) LAGE: 1..393
   (xi) SEQUENZBESCHREIBUNG: SEQ ID NO: 13:
   Met Arg Arg Gly Ile Ala Ala Thr Ala Leu Phe Ala Ala Val Ala Met
   1               5                   10                  15
   Thr Ala Ser Ala Cys Gly Gly Gly Asp Asn Gly Gly Ser Gly Thr Asp
               20                  25                  30
   Ala Gly Gly Thr Glu Leu Ser Gly Thr Val Thr Phe Trp Asp Thr Ser
           35                  40                  45
   Asn Glu Ala Glu Lys Ala Thr Tyr Gln Ala Leu Ala Glu Gly Phe Glu
       50                  55                  60
   Lys Glu His Pro Lys Val Asp Val Lys Tyr Val Asn Val Pro Phe Gly
   65                  70                  75                  80
   Glu Ala Asn Ala Lys Phe Lys Asn Ala Ala Gly Gly Asn Ser Gly Ala
                   85                  90                  95
   Pro Asp Val Met Arg Thr Glu Val Ala Trp Val Ala Asp Phe Ala Ser
               100                 105                 110
   Ile Gly Tyr Leu Ala Pro Leu Asp Gly Thr Pro Ala Leu Asp Asp Gly
           115                 120                 125
   Ser Asp His Leu Pro Gln Gly Gly Ser Thr Arg Tyr Glu Gly Lys Thr
       130                 135                 140
   Tyr Ala Val Pro Gln Val Ile Asp Thr Leu Ala Leu Phe Tyr Asn Lys
   145                 150                 155                 160
   Glu Leu Leu Thr Lys Ala Gly Val Glu Val Pro Gly Ser Leu Ala Glu
                   165                 170                 175
   Leu Lys Thr Ala Ala Ala Glu Ile Thr Glu Lys Thr Gly Ala Ser Gly
               180                 185                 190
   Leu Tyr Cys Gly Ala Thr Thr Arg Thr Trp Phe Leu Pro Tyr Leu Tyr
           195                 200                 205
   Gly Glu Gly Gly Asp Leu Val Asp Glu Lys Asn Lys Thr Val Thr Val
       210                 215                 220
   Asp Asp Glu Ala Gly Val Arg Ala Tyr Arg Val Ile Lys Asp Leu Val
   225                 230                 235                 240
   Asp Ser Lys Ala Ala Ile Thr Asp Ala Ser Asp Gly Trp Asn Asn Met
                   245                 250                 255
   Gln Asn Ala Phe Lys Ser Gly Lys Val Ala Met Met Val Asn Gly Pro
               260                 265                 270
   Trp Ala Ile Glu Asp Val Lys Ala Gly Ala Arg Phe Lys Asp Ala Gly
           275                 280                 285
   Asn Leu Gly Val Ala Pro Val Pro Ala Gly Ser Ala Gly Gln Gly Ser
       290                 295                 300
   Pro Gln Gly Gly Trp Asn Leu Ser Val Tyr Ala Gly Ser Lys Asn Leu
   305                 310                 315                 320
   Asp Ala Ser Tyr Ala Phe Val Lys Tyr Met Ser Ser Ala Lys Val Gln
   325 330 335
   Gln Gln Thr Thr Glu Lys Leu Ser Leu Leu Pro Thr Arg Thr Ser Val
   340 345 350
   Tyr Glu Val Pro Ser Val Ala Asp Asn Glu Met Val Lys Phe Phe Lys
   355 360 365
   Pro Ala Val Asp Lys Ala Val Glu Arg Pro Trp Ile Ala Glu Gly Asn
   370 375 380
   Ala Leu Phe Glu Pro Ile Arg Leu Gln
   385 390

## Beschreibung

Die vorliegende Erfindung bezieht sich auf die Isolierung von Genen, welche für Biosynthese-Enzyme von α-Amylase Hemmstoffen, sogenannten Pseudo-Oligosacchariden, kodieren. Insbesondere geht es dabei um Gene aus dem Streptomyceten Stamm Streptomyces glaucescens GLA.O (DSM 40716). Darüber hinaus wird die Verwendung dieser Gene zur Produktion von Acarbose und homologen Substanzen mit Streptomyces glaucescens (DSM 40716) deren heterologe Expression in anderen Produzentenstämmen von Pseudo-Oligosacchariden (z.B. Actinoplanes sp SE50/100) zur Produktionssteigerung und Produktionsstabilisierung, sowie deren heterologe Expression in anderen Mikroorganismen wie z.B. E. coli, Bacillus subtilis, Actinomycetales, wie z.B. Streptomyces-, Actinoplanes-, Ampullariella-, Streptoporangium-Stämmen, Streptomyces hygroscopicus var. limoneus, Streptomyces glaucescens, sowie biotechnologisch relevanten Pilzen (z.B. Aspergillus niger, Penicillium chrysogenum) und Hefen (z.B. Saccharomyces cerevisiae) beschrieben. Die Erfindung bezieht sich auch auf homologe Gene in anderen Mikroorganismen sowie auf Verfahren zu deren Isolierung.

Streptomyces glaucescens (DSM 40716) welches in der Literatur auch als Streptomyces glaucescens GLA.O bereichnet wird produziert die beiden Antibiotika Hydroxystreptomycin (Hütter (1967) Systematik der Streptomyceten. Basel Karger Verlag) und Tetracenomycin (Weber et al. (1979) Arch. Microbiol. 121: 111-116). Es ist bekannt, daß Streptomyceten zur Synthese strukturell verschiedener Naturstoffe befähigt sind. Oft sind aber die Bedingungen, unter denen diese Verbindungen produziert werden, nicht bekannt, oder die Substanzen werden nur in sehr geringen Mengen produziert und nicht detektiert.

Der α-Amylase-Hemmstoff Acarbose wurde aus verschiedenen Actinoplanes Stämmen (SE50, SE82 und SE18) isoliert (Schmidt et al. (1977) Naturwissenschaften 64: 535-536). Die Wirksubstanz wurde bei einem Screening nach α-Amylase-Hemmstoffen aus Organismen der Gattungen Actinoplanes, Ampullariella und Streptosporangium entdeckt. Bei der Acarbose handelt sich um ein Pseudotetrasaccharid, das aus einer ungewöhnlichen ungestättigten Cyclitol-Einheit besteht, an der ein Aminozucker 4,6 Dideoxy-4-Amino-D-glucopyranose gebunden ist. An dem Aminozucker können noch weitere α-1,4 glycosidisch gebundene D-Glucopyranose Einheiten gebunden sein. So enthält Acarbose z.B. zwei weitere Moleküle D-Glucose. Der Produzenten-Stamm synthetisiert ein Produktgemisch von Pseudo-Oligosacchariden, mit unterschiedlich langen Zuckerseitenketten (Schmidt et al. (1977) Naturwissenschaften 64: 535-536). Der Cyclitolrest der Acarbose ist identisch mit der Verbindung Valienamine, eine Komponente des Antibiotikums Validamycin A (Iwasa et al. (1979) J. Antibiot. 32: 595-602) aus Streptomyces hygroscopicus var. limoneus.

Acarbose kann fermentativ mit einem Actinoplanes Stamm hergestellt werden und hat als Therapeutikum für Diabetiker große wirtschaftliche Bedeutung erlangt. Actinoplanes synthetisiert ein Produktgemisch vonn α-Amylase Hemmstoffen, jedoch nur die Verbindung mit dem relativen Molekulargewicht von 645,5 (Acarviosin mit 2 Glucoseeinheiten (Truscheit (1984) VIIIth International Symposium on Medicinal Chemistry, Proc. Vol. I. Swedisch Academy of Pharmaceutical Sciences, Stockholm Sweden) wird unter dem generischen Namen Acarbose eingesetzt. Die Fermentationsbedingungen werden so gewählt, daß Acarbose das Hauptprodukt der Fermentation ist. Alternativ werden bestimmte Selektanten und Stämme verwendet, bei denen Acarbose entweder als Hauptprodukt entsteht, oder aber es werden Reinigungsverfahren zur selektiven Isolierung eingesetzt (Truscheit (1984) Vlllth International Symposium on Medicinal Chemistry, Proc. Vol. I. Swedisch Academy of Pharmaceutical Sciences, Stockholm Sweden). Möglich ist auch die chemische Umsetzung des Produktgemisches, um letztendlich das gewünschte Produkt Acarbose zu erhalten.

In der deutschen Patentanmeldung mit der Offenlegungsnummer 2 209 834 ist ein Verfahren zur Herstellung von Saccharose-Inhibitoren aus dem Actinoplanaceen-Stamm SE 50 beschrieben. Durch Stockmann und Piepersberg wurden Gensonden zur Detektion von 6-Desoxyhexose-Stoffwechsel in Streptomyceten, welche Sekundarmetabolite produzieren, beschrieben (FEMS Microbiology Letters 90, (1992), 185-190).

Im Gegensatz zur Gattung Streptomyces ist die Gattung Actinoplanes bislang genetisch noch nicht intensiv untersucht worden. Methoden, die für die Gattung Streptomyces etabliert wurden, sind nicht oder nicht immer auf die Gattung Actinoplanes übertragbar. Für eine gerichtete Optimierung der Acarboseproduktion mit molekularbiologischen Methoden müssen die Gene für die Acarbose-Biosynthese isoliert sowie charakterisiert werden. Dabei liegt es nahe zu versuchen, ein Wirts-Vektorsystem für Actinoplanes sp. zu etablieren. Dies ist jedoch aufgrund der wenig intensiven Untersuchungen von Actinoplanes sehr langwierig und aufwendig.

Die in der vorliegenden Patentanmeldung beschriebene Erfindung ist eine Lösung der Aufgabe, die Klonierung der Biosynthesegene für Acarbose und homologe Pseudo-Oligosaccharide vorzunehmen, und zwar aus Streptomyces glaucescens GLA.O, einem Streptomyceten, der genetisch gut untersucht ist (Crameri et al. (1983) J. Gen. Microbiol. 129: 519-527; Hintermann et al. (1984) Mol. Gen. Genet. 196: 513-520; Motamedi and Hutchinson (1987) PNAS USA 84: 4445-4449; Geistlich et al. (1989) Mol. Microbiol. 3: 1061-1069) und überraschenderweise ein Produzent von Acarbose ist. Streptomyces glaucescens GLA.O produziert in Stärke-haltigem Medium Pseudo-Oligosaccharide mit den Molekulargewichten 645, 807 und 970.

Ein Gegenstand der Erfindung ist also die Isolierung der entsprechenden Biosynthesegene aus Streptomyces glaucescens GLA.O und ihre Verwendung zur Isolierung der angrenzenden DNA Bereiche zur Komplettierung des Genclusters der genannten Biosynthesegene.

Die Isolierung der Gene für die Biosynthese von Pseudo-Oligosacchariden und deren Charakterisierung sind von großer Bedeutung, um die Biosynthese und deren Regulation besser verstehen zu können. Mit diesem Wissen kann dann die Produktivität des Stammes Streptomyces glaucescens GLA.O in bezug auf Acarbose-Produktion mit etablierten klassischen und molekularbiologischen Verfahren gesteigert werden. Darüberhinaus kann das gesamte Gencluster, das für die Synthese der Pseudo-Oligosaccharide kodiert, oder einzelne Gene davon auch in anderen biotechnologisch relevanten Mikroorganismen exprimiert werden, um eine weitere Produktionssteigerung oder eine Vereinfachung der Herstellung von Pseudo-Oligosacchariden wie z.B. Acarbose zu erreichen. Es kann auch durch gezielte Modifikation der Biosynthesegene ein Stamm hergestellt werden, der ausschließlich Acarbose mit einem Molekulargewicht von 645 produziert. Da die Gene für Antibiotikabiosynthesen immer geclustert vorliegen und of sehr stark konserviert sind (Stockmann und Piepersberg (1992) FEMS Microbiol. Letters 90: 185-190; Malpartida et al. (1987) Nature 314:642-644), können die Gene aus Streptomyces glaucescens GLA.O auch als Sonde zur Isolierung der Acarbose kodierenden Gene aus z.B. Actinoplanes sp. eingesetzt werden. Die Expression von Regulationsgenen oder von Genen, die für limitierende Biosyntheseschritte kodieren, kann in Streptomyces glaucescens GLA.O, Actinoplanes sp. oder entsprechenden Produzentenstämmen eine Steigerung der Produktivität zur Folge haben. Ebenfalls kann eine Steigerung der Produktivität durch eine Ausschaltung ("knock out" oder Mutagenese) von solchen Acarbose-Biosynthesegenen erzielt werden, die bei der Biosynthese hemmend wirken.

Eine mögliche Strategie zur Klonierung bis dahin noch nicht isolierter Antibiotika-Biosynthesegene besteht in der Verwendung genspezifischer Sonden (Stockmann und Piepersberg (1992) FEMS Microbiol. Letters 90: 185-190; Malpartida et al. (1987) Nature 314:642-644). Dies können entweder P³²-markierte oder anders markierte DNA-Fragmente sein oder aber man amplifiziert die entsprechenden Gene direkt aus den zu untersuchenden Stämmen mit degenerierten PCR-Primern und isolierter chromosomaler DNA als Template.

Das letztgenannte Verfahren wurde in der vorliegenden Arbeit eingesetzt. Pseudo-Oligosaccharide wie die Acarbose enthalten als Strukturelement einen 4,6-Deoxyglucose Baustein. Es ist bekannt, daß an der Biosynthese von 4,6-Deoxyglucose das Enzym dTDP-Glucose-4,6-Dehydratase beteiligt ist (Stockmann und Piepersberg (1992) FEMS Microbiol. Letters 90: 185-190). Da Deoxyzucker ein häufiger Bestandteil von Naturstoffen und Antibiotika sind, ist dieses Enzym möglicherweise ein Mittel zur Isolierung der entsprechenden Antibiotika-Biosynthesegene. Da diese immer als Cluster vorliegen, genügt zunächst die Isolierung eines Genes; anschließende kann dann die Isolierung und Charakterisierung der angrenzenden DNA-Bereiche vorgenommen werden.

Eine dTDP Glucose 4,6 Dehydratase katalysiert z.B. einen Schritt in der Biosynthese von Hydroxystreptomycin bei Streptomyces glaucescens GLA.O (Retzlaff et al. (1993) Industrial Microorganisms. Basic and applied molecular genetics ASM, Washington DC, USA). Weitere dTDP-Glucose 4,6-Dehydratasen sind aus anderen Mikroorganismen isoliert worden z. B. aus Streptomyces griseus (Pissowotzki et al. (1991) Mol. Gen. Genet. 231: 113-123), Streptomyces fradiae (Merson-Davies and Cundcliffe (1994) Mol. Microbiol. 13: 349-355) und Streptomyces violaceoruber (Bechthold, et al. (1995) Mol. Gen. Genet. 248: 610-620).

Die Sequenz der PCR Primer zur Amplifizierung einer dTDP-Glucose-4,6-Dehydratase konnte also von der Aminosäure-Sequenz bereits bekannter Biosynthesegene abgeleitet werden. Es wurden dazu konservierte Bereiche in der Proteinsequenz dieser Enzyme ausgewählt und die Aminosäuresequenz wurde entsprechend des genetischen Codes in eine Nukleinsäuresequenz übersetzt. Die Proteinsequenzen wurden aus den EMBL und Genbank, Datenbanken entnommen. Folgende Sequenzen wurden benutzt:Streptomyces griseus; accession number: X62567 Gen: strE (vom 30.10.1993); Streptomyces violaceoruber; accession number: L37334 Gen: graE (vom 10.04.1995); Saccharopolyspora etythraea; accession number: L37354 Gen: gdh (vom 09.11.1994). Es ergeben sich auf Grund der Degeneriertheit des genetischen Codes viele mögliche Primersequenzen. Da Streptomyceten in der dritten Position eines Codons meist ein G oder C enthalten (Wright and Bibb (1992) Gene 113: 55-65), reduziert sich die Anzahl der zu synthetisierenden Primer. Mit diesen Primergemischen kann dann eine PCR Amplifikation mit der DNA von zu untersuchenden Stämmen durchgeführt werden, die im Idealfall zu einem amplifizierten DNA-Fragment führt. Dieses Fragment weist bei hochkonservierten Proteinen eine vorhersehbare Länge auf, die sich durch den Abstand der Primer in der Nukleinsäuresequenz des entsprechenden Genes voneinander ergeben. Ein solcher Versuchsansatz muß aber nicht zwangsläufig zu einem Amplifikat führen. Die Primer können entweder zu unspezifisch sein und sehr viele Fragmente amplifizieren, oder aber man erhält kein PCR-Produkt, wenn es für die hergestellten PCR-Primer keine komplementäre Bindungsstellen im Chromosom gibt.

Die Untersuchung des Streptomyceten-Stammes Streptomyces glaucescens GLA.0 erbrachte ein amplifiziertes DNA Fragment (acbD*) mit der erwarteten Länge von 550 bp. Die weitere Untersuchung ergab, daß dieser Stamm neben einem dTDP Glucose-4, 6-Dehydratase Gen für die Biosynthese von Hydroxystreptomycin überraschenderweise ein zweites dTDP Glucose-4, 6-Dehydratase Gen für die Biosynthese von Pseudo-Oligosacchariden wie der Acarbose enthält. Die beiden Gene weisen eine hohe Homologie auf, sind aber auf Aminosäureebene lediglich zu 65% identisch.

Mit der Sonde acbD* (siehe Beispiel 2 und Tabelle 2A) wurde ein 6,8 kb langes Pstl-DNA Fragment aus Streptomyces glaucescens GLA.O isoliert, das für verschiedene Gene kodiert (acbA, acbB, acbC, acbD, acbE, acbF), die an der Biosynthese der Pseudo-Oligosaccharide beteiligt sind.

Durch Deletion der Gene acbBCD (Aminotransferase acbB, dTDP-Glucose Synthase acbC, dTDP Glucose-4,6-Dehydratase acbD, siehe Beispiel 6) wurde eine Mutante von Streptomyces glaucescens GLA.O erzeugt, die in dem Produktionsmedium keine Pseudo-Oligosaccharide mehr produziert. Die Beteiligung der Gene acbBCD an der Synthese von Pseudo-Oligosacchariden ist also durch die Deletion der entsprechenden Loci belegt worden.

Die beiden Gene dTDP-Glucose Synthase und dTDP-Glucose-4,6-Dehydratase dürften an der Biosynthese des Deoxyzuckers der Pseudo-Oligosaccharide beteiligt sein, was aus der Funktion gut untersuchter homologer Enzyme (siehe oben) zu schließen ist. Die Aminotransferase (kodiert durch das acbB Gen) ist wahrscheinlich für die Übertragung der Aminogruppe entweder auf den Zucker- oder den Cyclitolrest verantwortlich. Durch die Analyse der Proteinsequenz von acbB konnte ein Aminosäuremotif gefunden werden, daß an der Bindung von Pyridoxal-Phosphat beteiligt ist. Dieses Motif ist typisch für Aminotransferasen der Klasse III (EC 2.6.1.11; EC 2.6.1.13; EC 2.6.1.18; EC 2.6.1.19; EC 2.6.1.62; EC 2.6.1.64; EC 5.4.3.8). Die exakte enzymatische Funktion von acbB kann erst bei der weiteren Untersuchung der Biosynthese der Pseudo-Oligosaccharide geklärt werden. AcbE kodiert für ein Transkriptions-Regulationsprotein mit großen Ähnlichkeiten zu DNAbindenden Proteinen mit einem Helix-Turn-Helix Motif auf (z.B. Bacillus subtilis DegA, P37947: Swiss-Prot Datenbank). So hemmt der Transkriptions-Aktivator CcpA aus Bacillus subtilis die Bildung von α-Amylase bei Anwesenheit von z.B. Glucose (Henkin et al. (1991) Mol. Microbiol. 5: 575-584). Bei anderen Vetretem dieser Gruppe handelt es sich um Proteine, die bestimmte Zuckerbausteine erkennen und einen positiven oder negativen Effekt auf die Biosynthese von Stoffwechselwegen aufweisen können. Auch die Biosynthese der Pseudo-Oligosaccharide in Streptomyces glaucescens GLA.O wird reguliert. Die Synthese der Pseudo-Oligosaccharide konnte bisher nur auf Stärke-haltigen Medien nachgewiesen werden. AcbE könnte demnach für die Regulation der Pseudo-Oligosaccharid-Synthese verantwortlich sein, der genaue Mechanismus ist aber noch nicht bekannt. Das Gen kann aber jetzt mit molekularbiologischen Methoden gezielt modifiziert werden, um eine gesteigerte Pseudo-Oligosaccharid-Biosyntheserate zu erzielen. Desweiteren kann die DNA Bindestelle von acbE durch sogenannte Gelshift-Assays identifiziert werden (Miwa et al. (1994) Microbiology 140: 2567-2575). Nach Identifikation und anschließender Modifikation der Promotoren und anderer regulatorischer DNA-Regionen, welche für die Transkription der Pseudo-Oligosaccharid-Gene verantwortlich sind, kann eine Steigerung der Biosyntheserate von Acarbose erreicht werden.

Die Funktion von acbF ist derzeit noch nicht eindeutig bekannt. Das entsprechende Genprodukt weist Homologien zu Zucker-bindenden Proteinen, wie zum Beispiel dem Zucker-Bindeprotein aus Streptococcus mutans auf (MsmE; Q00749: Swissprot Datenbank), was eine Beteiligung an der Biosynthese der Pseudo-Oligosaccharide wahrscheinlich macht. Das Genprodukt des acbA Gens weist Homologien zu bekannten bakteriellen ATP-Bindeproteinen auf (z.B. aus Streptomyces peucitus DrrA, P32010: SwissProt Datenbank). Das AcbA Protein weist das typische ATP/GTP Bindungsmotif, den sogenannten P-Loop, auf. Diese Proteine stellen einen wichtigen Bestandteil von sogenannten ABC-Transportern dar, die am aktiven Transport von Metaboliten an biologischen Membranen beteiligt sind (Higgins (1995) Cell 82: 693-696). AcbA könnte demnach für den Export der Pseudo-Oligosaccharide aus der Zelle verantwortlich sein oder aber am Import von Zuckerbausteinen für die Biosynthese von α-Amylasehemmstoffen wie z.B. Maltose beteiligt sein.

Alle bisher analysierten Biosynthesegene für Sekundärmetaboliten aus Streptomyceten sind in einem Cluster angeordnet. Deswegen ist ein solches Gencluster auch für die anmeldungsgemäßen Acarbose-Biosynthesegene anzunehmen ist. Somit können durch Isolierung der angrenzenden DNA Bereiche des erfindungsgemäßen 6,8 kb Pstl DNA Fragmentes auch die restlichen Gene, die relevant für die Pseudo-Oligosaccharid Biosynthese sind, isoliert werden. Wie auch schon oben erwähnt, ist die Isolierung von homologen Genclustern aus Mikroorganismen verschieden von Streptomyces glaucescens (DSM 40716), leicht möglich.

Gegenstand der Erfindung ist daher ein rekombinantes DNA-Molekül, enthaltend Gene für die Biosynthese von Acarbose, welches
(a) eine DNA-Sequenz gemäß Tabelle 4 enthält;
(b) eine DNA-Sequenz enthält, die in der Lage ist, mit dem DNA-Molekül gemäß

(a) unter stringenten Bedingungen zu hybridisieren ; oder
(c) eine DNA-Sequenz enthält, die aufgrund der Degeneriertheit des genetischen Codes von den DNA-Molekülen gemäß (a) und (b) verschieden ist, jedoch die Expression der entsprechend mit dem DNA-Molekül gemäß (a) und (b) exprimierbaren Proteinen ermöglicht.

Desweiteren sind Gegenstände der Erfindung ein rekombinantes DNA-Molekül, enthaltend das acbA-Gen, insbesondere dadurch gekennzeichnet, daß es die DNA-Sequenz der Nukleotide 1 bis 720 nach Tabelle 4 oder Teile davon enthält; ein rekombinantes DNA-Molekül, enthaltend das acbB-Gen, insbesondere dadurch gekennzeichnet, daß es die DNA-Sequenz der Nukleotide 720 bis 2006 nach Tabelle 4 oder Teile davon enthält; ein rekombinantes DNA-Molekül, enthaltend das acbC-Gen, insbesondere dadurch gekennzeichnet, daß es die DNA-Sequenz der Nukleotide 2268 bis 3332 nach Tabelle 4 oder Teile davon enthält; ein rekombinantes DNA-Molekül, enthaltend das acbD-Gen, insbesondere dadurch gekennzeichnet, daß es die DNA-Sequenz der Nukleotide 3332 bis 4306 nach Tabelle 4 oder Teile davon enthält; ein rekombinantes DNA-Molekül, enthaltend das acbE-Gen, insbesondere dadurch gekennzeichnet, daß es die DNA-Sequenz der Nukleotide 4380 bis 5414 nach Tabelle 4 oder Teile davon enthält; ein rekombinantes DNA-Molekül, enthaltend das acbF-Gen, insbesondere dadurch gekennzeichnet, daß es die DNA-Sequenz der Nukleotide 5676 bis 6854 nach Tabelle 4 oder Teile davon enthält.

Weitere Gegenstände der Erfindung ist ein Oligonukleotid-Primer zur PCR-Amplifizierung eines rekombinanten DNA-Moleküls wie oben beschrieben, enthaltend Gene für die Biosynthese von Acarbose, wobei der Primer insbesondere die Sequenz gemäß Tabelle 1 hat.

Ein weiterer Gegenstand der Erfindung ist ein Vektor, enthaltend ein rekombinantes DNA-Molekül, welches ein DNA-Molekül wie im vorletzten und vorvorletzten Absatz beschrieben enthält, insbesondere dadurch gekennzeichnet, daß der Vektor ein Expressionsvektor ist und das besagte DNA-Molekül operativ mit einer Promotersequenz verbunden ist, wobei der Vektor vorzugsweise geeignet ist zur Expression in Wirtsorganismen, ausgewählt aus der Gruppe bestehend aus E. coli, Bacillus subtilis, Actinomycetales, wie z.B. Streptomyces-, Actinoplanes-, Ampullariella-, Streptosporangium-Stämmen, Streptomyces hygroscopicus var. limoneus, Streptomyces glaucescens, sowie biotechnologisch relevanten Pilzen (z.B. Aspergillus niger, Penicillium chrysogenum) und Hefen (z.B. Saccharomyces cerevisiae), ganz besonders bevorzugt Streptomyces glaucescens (DSM 40716) oder Actinoplanes sp. ist. Da die operative Verbindung des besagten DNA-Moleküls mit Promotersequenzen des Vektors nur eine vorzugsweise Ausführungsform der Erfindung ist, ist auch eine Expression mittels in bezug auf das DNA-Molekül endogener Promotorsequenzen , z.B. der jeweils natürlichen oder in bezug auf die Optimierung der Acarbose-Ausbeute mutierten natürlichen Promotoren möglich. Solche natürlichen Promotoren sind Teil des erfindungsgemäßen DNA-Moleküls.

Ein weiter Gegenstand der Erfindung ist ein Vektor enthaltend ein erfindungsgemäßes DNA-Molekül zur Verwendung in einem Verfahren zur Ausschaltung oder Veränderung von natürlichen Acarbose-Biosynthesegenen in einem Acarbose-produzierenden Mikroorganismus. Ein solcher Vektor wird vorzugsweise ausgewählt aus der Gruppe enthaltend pGM160 und Vektoren wie in den Europäischen Patenten EP 0 334 282 und EP 0 158 872 beschrieben.

Ein weiterer Gegenstand der Erfindung ist eine Wirtszelle, transformiert mit einem der oben beschriebenen DNA-Moleküle oder Vektoren, insbesondere dadurch gekennzeichnet, daß besagte Wirtszelle aus der Gruppe bestehend aus E. coli, Bacillus subtilis, Actinomycetales, wie z.B. Streptomyces-, Actinoplanes-, Ampullariella-, Streptosporangium-Stämmen, Streptomyces hygroscopicus var. limoneus, Streptomyces glaucescens, sowie biotechnologisch relevanten Pilzen (z.B. Aspergillus niger, Penicillium chrysogenum) und Hefen (z.B. Saccharomyces cerevisiae) ausgewählt wird; ganz besonders bevorzugt, daß sie aus der Gruppe bestehend aus Streptomyces glaucescens (DSM 40716) und Actinoplanes sp. ausgewählt wird.

Ein weiterer Gegenstand der Erfindung sind isolierte Proteine, erhältlich durch Expression der Gene, welche von dem im vorigen Absatz beschriebenen DNA-Molekül kodiert werden.

Die folgenden Aussagen gelten für alle im Rahmen der vorliegenden Erfindung identifizierten Einzelgene und sind nur aus Gründen der Übersichtlichkeit zusammengefaßt worden: Weiterer Gegenstand der Erfindung ist ein Protein, kodiert von einem rekombinanten DNA-Molekül wie im vorletzten Absatz beschrieben, insbesondere dadurch gekennzeichnet, daß es die DNA-Sequenz der Nukleotide 1 bis 720 bzw. 720 bis 2006 bzw. 2268 bis 3332 bzw. 3332 bis 4306 bzw. 4380 bis 5414 bzw. 5676 bis 6854 nach Tabelle 4 oder Teile davon enthält; ganz besonders bevorzugt ein Protein, kodiert vom acbA-Gen bzw. acbB-Gen bzw. acbC-Gen bzw. acbD-Gen bzw. acbE-Gen bzw. acbF-Gen, enthaltend die Aminosäuresequenz gemäß Tabelle 4.

Ein weiterer Gegenstand der Erfindung ist ein Verfahren zur Gewinnung der Proteine, die im Vorhergehenden als Gegenstände der Erfindung beschrieben wurden, dadurch gekennzeichnet, daß
(a) die Proteine in einer geeigneten Wirtszelle, insbesondere dadurch gekennzeichnet, daß besagte Wirtszelle aus der Gruppe bestehend aus E. coli, Bacillus subtilis, Actinomycetales, wie z.B. Streptomyces-, Actinoplanes-, Ampullariella-, Streptosporangium-Stämmen, Streptomyces, hygroscopicus var. limoneus, Streptomyces glaucescens, sowie biotechnologisch relevanten Pilzen (z.B. Aspergillus niger, Penicillium chrysogenum) und Hefen (z.B. Saccharomyces cerevisiae) ausgewählt wird; ganz besonders bevorzugt, daß sie aus der Gruppe bestehend aus Streptomyces glaucescens (DSM 40716) und Actinoplanes sp. ausgewählt wird, exprimiert und
(b) isoliert werden.

Ein weiterer Gegenstand der Erfindung ist ein Verfahren zur Herstellung von Acarbose, dadurch gekennzeichnet, daß
(a) eines oder mehrere Gene des rekombinanten DNA-Moleküls, welches eine DNA-Sequenz gemäß Tabelle 4 oder Teile davon enthält oder eine DNA-Sequenz enthält, die in der Lage ist, mit dem DNA-Molekül gemäß Tabelle 4 unter stringenten Bedingungen zu hybridisieren, oder Teile davon, oder eine DNA-Sequenz enthält, die aufgrund der Degeneriertheit des genetischen Codes von den gerade beschriebenen DNA-Molekülen verschieden ist, jedoch die Expression der entsprechend mit diesen DNA-Molekülen exprimierbaren Proteinen ermöglicht, oder Teile davon, zur Expression in einer geeigneten Wirtszelle, die insbesondere aus dergleichen Gruppe wie im letzten Absatz beschrieben, ausgewählt wird, benutzt werden und
(b) die Acarbose aus Kulturüberständen besagter Wirtszelle isoliert wird.

Ein weiterer Gegenstand der Erfindung ist ein Verfahren zur Herstellung von Acarbose, dadurch gekennzeichnet, daß
(a) eines oder mehrere Gene des rekombinanten DNA-Moleküls, welches eine DNA-Sequenz gemäß Tabelle 4 oder Teile davon enthält oder eine DNA-Sequenz enthält, die in der Lage ist, mit dem DNA-Molekül gemäß Tabelle 4 unter stringenten Bedingungen zu hybridisieren, oder Teile davon, oder eine DNA-Sequenz enthält, die aufgrund der Degeneriertheit des genetischen Codes von den gerade beschriebenen DNA-Molekülen verschieden ist, jedoch die Expression der entsprechend mit diesen DNA-Molekülen exprimierbaren Proteinen ermöglicht, oder Teile davon, in einer acarboseproduzierenden Wirtszelle, insbesondere Streptomyces glaucescens (DSM 40716) und Actinoplanes sp., ausgeschaltet werden und
(b) die Acarbose aus besagter Wirtszelle isoliert wird.

Die Ausschaltung eines oder mehrere Gene kann dabei nach molekularbiologischen Standardmethoden, z.B. unter Benutzung der oben beschriebenen Vektoren (pGM160 und andere), vorgenommen werden. Ein auszuschaltendes Gen könnte z.B. das acbE-Gen sein, welches wahrscheinlich eine Regulationsfunktion hat. Ebenfalls könnte man Gene mit dem Ziel ausschalten, nur noch reine Acarbose als Fermentationsprodukt zu erhalten und kein Gemisch homologer Pseudo-Oligosaccharide mehr (s.o.). Vorzugsweise dienen zur Ausschaltung von besagten Genen die oben für diesen Zweck beschriebenen Vektoren.

Ein weiterer Gegenstand der Erfindung ist ein Verfahren zur Herstellung von Acarbose, dadurch gekennzeichnet, daß die in den vorigen beiden Absätzen beschriebenen Verfahren zur Herstellung von Acarbose miteinander kombiniert werden, daß also ein oder mehrere der besagten Gene künstlich exprimiert und ein oder mehrere der besagten Gene ausgeschaltet werden.

Ein weiterer Gegenstand der Erfindung ist ein Verfahren zur Veränderung der Genexpression von endogenen Acarbose-Biosynthesegenen durch Mutierung des jeweiligen Genpromotors, um verbesserte Acarbose-Ausbeuten zu erhalten. Die Mutationen können dabei nach bekannten homologen Rekombinationsverfahren in den zu verbessernden Produktionsstamm eingebracht werden. Solche Mutationen können Transitionen, Deletionen und/oder Additionen sein. Eine "Addition" kann z.B. das Hinzufügen eines einzigen oder mehreren Nukleotiden bedeuten oder einer oder mehrerer positiv regulatorisch wirkenden DNA-Sequenz bedeuten, die eine Verstärkung der Expression eines endogenen Biosynthesegens für Acarbose bewirkt. Auch der umgekehrte Fall, das Hinzufügen einer negativ regulatorisch wirkenden DNA-Sequenz zur Repression eines endogenen Acarbose-Biosynthesegens ist eine vorzugsweise Form einer Addition. "Transitionen" können z.B. Nukleotidaustausche sein, die negativ oder positiv regulatorische Elemente in ihrer Wirkung abschwächen oder verstärken. Durch "Deletionen" können negativ oder positiv regulatorische Elemente entfernt werden. Die endogenen Gene dieses Verfahrens liegen vorzugsweise in Actinomycetales, wie z.B. Streptomyces-, Actinoplanes-, Ampullariella-, Streptosporangium-Stämmen, Streptomyces hygroscopicus var. limoneus, Streptomyces glaucescens vor; ganz besonders liegen sie in Streptomyces glaucescens (DSM 40716) und Actinoplanes sp. vor.

Ein weiterer Gegenstand der Erfindung ist die Verwendung von Streptomyces (DSM 40716) zur Gewinnung von Acarbose.

Ein weiterer Gegenstand der Erfindung ist die Verwendung von Streptomyces (DSM 40716) zur Herstellung von Mutanten dieses Stammes auf "klassischem Wege", die eine ertragreichere Acarboseproduktion ermöglichen. Die Herstellverfahren für solche verbesserten Naturstoffproduzenten sind seit langem bekannt und bedienen sich häufig klassischen Mutagenese- und Selektionsschritten.

Ein weiterer Gegenstand der Erfindung ist ein Verfahren zur Vervollständigung des Genclusters für die Biosynthese von Acarbose und homologen Polysacchariden gemäß Tabelle 4, dadurch gekennzeichnet, daß
a) Hybridisierungsproben hergestellt werden, die von dem DNA-Molekül gemäß Tabelle 4 abgeleitet werden,
b) diese Hybridierungsproben zum genomischen Screening von DNA-Bibliotheken, erhalten aus Streptomyces glaucescens (DSM 40716), unter stringenten Bedingungen eingesetzt werden, und
c) die gefundenen Klone isoliert und charakterisiert werden.

Ein weiterer Gegenstand der Erfindung ist ein Verfahren zur Vervollständigung des Genclusters für die Biosynthese von Acarbose und homologen Pseudo-Oligosacchariden gemäß Tabelle 4, dadurch gekennzeichnet, daß ausgehend von dem rekombinanten DNA-Molekül gemäß Tabelle 4
a) PCR-Primer hergestellt werden,
b) diese PCR-Primer zur Anreicherung von DNA-Fragmenten von genomischer DNA von Streptomyces glaucescens (DSM 40716) benutzt werden, wobei diese Primer mit solchen Primem kombiniert werden, die von Sequenzen des verwendeten Vektorsystems hybridisieren,
c) die angereicherten Fragmente isoliert und charakterisiert werden.

Ein weiterer Gegenstand der Erfindung ist ein Verfahren zur Isolierung eines Genclusters für die Biosyntnese von Acarbose und homologen Pseudo-Oligosacchariden aus Acarbose-produzierenden Mikroorganismen, die verschieden von Streptomyces glaucescens (DSM 40716) sind, dadurch gekennzeichnet, daß ausgehend von dem rekombinanten DNA-Molekül gemäß Anspruch 4
a) Hybridisierungsproben hergestellt werden,
b) diese Hybridierungsproben zum genomischen oder cDNA-Screening von DNA-Bibliotheken, erhalten aus dem entsprechenden Mikroorganismus, unter stringenten Bedingungen eingesetzt werden, und
c) die gefundenen Klone isoliert und charakterisiert werden.

Ein weiterer Gegenstand der Erfindung ist ein Verfahren zur Isolierung eines Genclusters für die Biosynthese von Acarbose und homologen Pseudo-Oligosacchariden aus Acarbose produzierenden Mikroorganismen, die verschieden von Streptomyces glaucescens (DSM 40716) sind, dadurch gekennzeichnet, daß ausgehend von dem rekombinanten DNA-Molekül gemäß Anspruch 4
a) PCR-Primer hergestellt werden,
b) diese PCR-Primer zur Anreicherung von DNA-Fragmenten von genomischer DNA oder cDNA eines entsprechenden Mikroorganismus benutzt werden,
c) die angereicherten Fragmente isoliert und charakterisiert werden und
d) ggf. in einem Verfahren wie im vorigen Absatz beschrieben eingesetzt werden.

Die beschriebenen Verfahren zur Isolierung eines Genclusters für die Biosynthese von Acarbose und homologen Pseudo-Oligosacchariden aus Acarbose produzierenden Mikroorganismen, die verschieden von Streptomyces glaucescens (DSM 40716), sind dadurch gekennzeichnet, daß die Mikroorganismen aus der Gruppe bestehend aus Actinomycetales, wie z.B. Streptomyces-, Actinoplanes-, Ampullariella-, Streptosporangium-Stämmen, Streptomyces hygroscopicus var. limoneus, Streptomyces glaucescens ausgewählt werden, vorzugweise aus der Gruppe, bestehend aus Streptomyces glaucescense (DSM 40716) und Actinoplanes-sp. ausgewählt werden.

Ein weiterer Gegenstand der Erfindung ist die Verwendung von Streptomyces glaucescens (DSM 40716) zur Isolierung von Acarbose.

Die Erfindung wird nun anhand der Beispiele, Tabellen und Figuren näher erläutert, ohne darauf beschränkt zu sein.

Alle Plasmidisolierungen wurden mit einen Isolierungskit (Nucleobond^{®}) der Firma Macherey und Nagel (Düren, Deutschland) nach Angaben des Herstellers durchgeführt. Molekularbiologische Arbeitsschritte wurden nach Standardprotokollen (Sambrock et al. (1989) Molecular cloning: A Laboratory Manual, 2nd ed. Cold Spring Harbor Laboratory, USA) oder nach Angaben des jeweiligen Herstellers durchgeführt. DNA- und Proteinsequenzen wurden mit der Genetics Computer Group Software, Version 8 untersucht (Programme: FastA, TFastA, BlastX, Motifs, GAP, CODONPREFERENCE) und den Datenbanken Swiss-Prot (Release 32), EMBL (Release 46) und Prosite (Release 12.2). Die molekularbiologische Bearbeitung von Streptomyces glaucescens und Actinoplanes (DNA-Isolierung, DNA-Transformationen) wurden wie in Hopwood et al.:Genetic Manipulation of Streptomycetes: A Laboratory Manual. The John Innes Foundation, Norwich, UK, 1985 und Motamedi and Hutchinson: Cloning and heterologous expression of a gene cluster for the biosynthesis of tetracenomycin C, the anthracycline antitumor antibiotic of Streptomyces glaucescens. Proc. Natl. Acad. Sci. USA 84:4445-4449 (1987), beschrieben durchgeführt.

Hybridisierungen wurden generell mit dem "Non radioactive DNA labeling kit" von Boehringer Mannheim (Cat. No. 1175033) vorgenommen. Die Visualisierung erfolgte mit dem "Luminescent Detection Kit" von Boehringer Mannheim (Cat. No. 1363514). Die Hybridisierung erfolgte in allen in dieser Patentanmeldung angegebenen Beispielen unter stringenten Bedingungen: 68°C, 16 Std. 5xSSC, 0,1 % N-Laurylsarkosin, 0,02 % SDS, 1 % Blocking Reagent (Boehringer Mannheim). SSC bedeutet 0,15 M NaCl/0,015 M Natriumcitrat. Die hier gegebene Definition von "stringenten Bedingungen" gilt für alle Aspekte der vorliegenden Anmeldung, die sich auf "stringente Bedingungen" beziehen. Dabei soll die Art der Erreichung dieser Stringenz, d.h. die angegebenen Hybridisierungsbedingungen, nicht beschränkend wirken, da der Fachmann auch andere Bedingungen wählen kann, um gleiche stringente Bedingungen zu erzeugen, z.B. durch andere Hybridisierungslösungen in Verbindung mit anderen Temperaturen.

### Beispiel 1: Synthese und Sequenz der PCR Primer und Amplifikation der Fragmente aus S. glaucescens (DSM 40716)

Die PCR erfolgte nach Standardbedingungen mit jeweils 100 pmol Primer 1 und 2 in 100 *µ*l Reaktionsansatz

| | |
|---|---|
| PCR Puffer¹ | 10*µ*l |
| PCR Primer | jeweils 2,5 *µ*l |
| dNTPs | jeweils 0,2 mM |
| BSA (10 mg/ml) | 1 µl |
| Template DNA | 1 µg (1 µl) |
| Taq Polymerase² (5 units/ml) | 1,5 µl |
| H₂O | auf 100 µl auffüllen |
| ¹: Promega | |
| ²: Boehringer Mannheim | |

Die Proben werden mit 75 *µ*l Mineralöl überschichtet und die Amplifikation mit einem DNA Thermal Cyler TC1 der Firma Perkin Elmer durchgeführt.

### Parameter:

| Zyklen | Temperatur | Dauer |
|---|---|---|
| 1 | 96°C | 5 min |
| | 74°C | 5 min |
| 30 | 95°C | 1,5 min |
| | 74°C | 1,5 min |
| 1 | 74°C | 5 min |

In Tabelle 1 ist die Sequenz der degenerierten Primer aufgeführt, die zur Amplifikation von dTDP-Glucose-Dehydratasen aus verschiedenen Streptomyceten verwendet werden sollte.

**Tabelle 1: Primersequenzen zur Amplifiaktion von dTDP-glucose-4,6-dehydratasen**

| | | |
|---|---|---|
| Primer 1: | CSGGSGSSGCSGGSTTCATSGG | (SEQ ID NO.: 1) |
| Primer 2: | GGGWVCTGGYVSGGSCCGTAGTTG | (SEQ ID NO.: 2) |

Hierbei bedeuten S=G oder C, W=A oder T, V=A oder G, Y=T oder C.

### Beispiel 2: DNA Sequenz der isolierten PCR-Fragmente aus Streptomyces glaucescens (DSM 40716)

Die Sequenzierung erfolgte nach der Didesoxy-Strangabruch Methode nach Sanger et al. (PNAS USA, 74: 5463-5467 (1977)). Die Reaktionen wurden mit dem Auto Read Sequenzing Kit^{®} der Firma Pharmacia Biotech (Freiburg, Deutschland) nach Angaben des Herstellers durchgeführt. Die Auftrennung und Detektion erfolgte über einen ALF DNA Sequencer^{®} der Firma Pharmacia Biotech (Freiburg, Deutschland).

Die anschließende Klonierung der PCR-Fragmente (Sure Clone Kit^{®}, Pharmacia Biotech, Freiburg) in den E. coli Vektor pUC18 und die Sequenzierung des Fragmentes belegte die Vermutung, daß es sich um eine dTDP-Glucose-4,6-Dehydratase handelt. Allerdings wurden 2 verschiedene Gene isoliert, die beide hohe Homologien zu dTDP-Glucose-4,6-Dehydratase aufweisen, jedoch nicht identisch sind. Die PCR-Fragmente werden im folgenden als acbD* und HstrE* bezeichnet.

Die Sequenz der isolierten PCR Fragmente ist in Tabelle 2A und 2B gezeigt und der Homologievergleich der abgeleiteten Aminosäuresequenz von HstrE* und acbD* in Tabelle 2C. Die beiden Proteine weisen eine Identität von lediglich 65% auf.

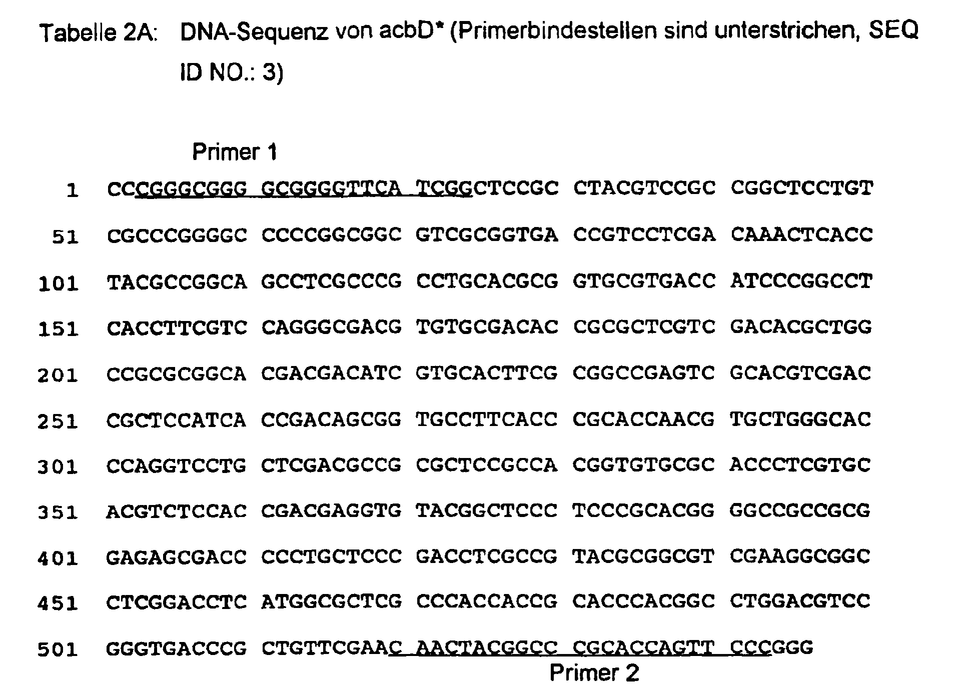

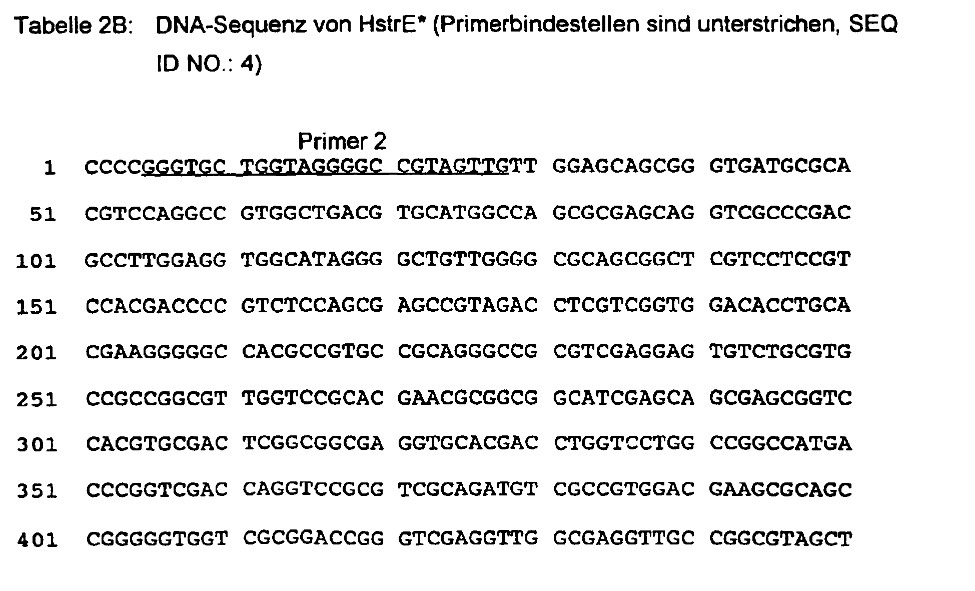

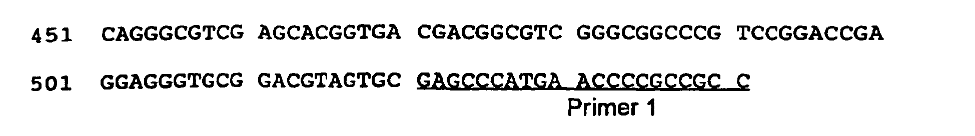

**Tabelle 2C: Homologie Vergleich der abgeleiteten Aminosäuresequenz der PCR-Produkte HstrE* und acbD* (Programm: GAP)**

| | | | |
|---|---|---|---|
| Qualität: | 196.3 | Länge: | 182 |
| Verhältnis: | 1.091 | Lücken: | 0 |
| Prozent Ähnlichkeit: | 77.654 | Prozent Identität: | 65.363 |

### PCRstrE.Pep x PCRacbD.Pep

jeweils obere Reihe: SEQ ID NO.: 5
jeweils untere Reihe: SEQ ID NO.: 6

### Beispiel 3: Southern-Analyse mit chromosomaler DNA aus Streptomyces glaucescens (DSM 40716) und den isolierten und markierten PCR-Fragmenten

Die Zellen wurden in R2YENG Medium angezogen und nach 30 h für die DNA Isolierung geerntet. Die Isolierung der chromosomalen DNA aus S. glaucescens (DSM 40716) wurde wie bei Hopwood et al. ((1985) Genetic manipulations of Streptomyces: a laboratory manual. The John Innes Foundation, Norwich UK) beschrieben, durchgeführt.

Mit den markierten Sonden des PCR-Fragmentes acbD* und HstrE* wurde eine Southern Blot Analyse mit chromosomaler DNA des Produzentenstammes S. glaucescens (DSM 40716) durchgeführt, die mit Pstl, BgIII und BamHI verdaut wurde. Die beiden PCR-Fragmente wurden mit Digoxygenin nach Angaben des Herstellers (Boehringer Mannheim; Mannheim) markiert und ein Verdau chromosomaler DNA des Produzenten-Stammes Streptomyces glaucescens (DSM 40716) auf einem Agarosegel aufgetrennt. Die DNA wurde durch Kapillartransfer auf Nylonmembranen übertragen und anschließend homologe DNA Bereiche mit den markierten Sonden nach Hybridisierung sichtbar gemacht.

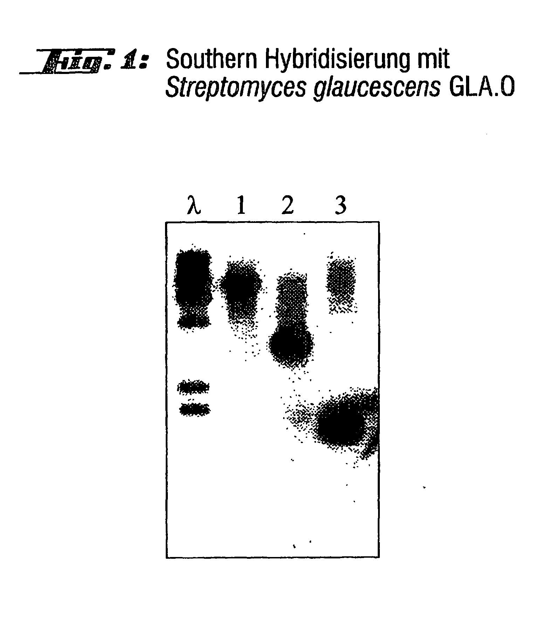

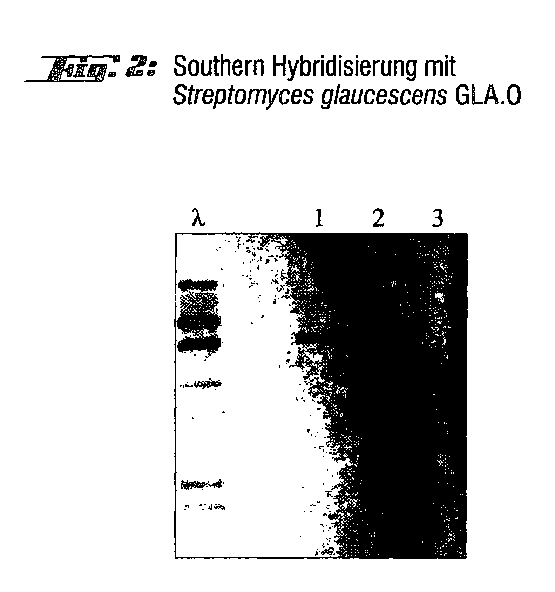

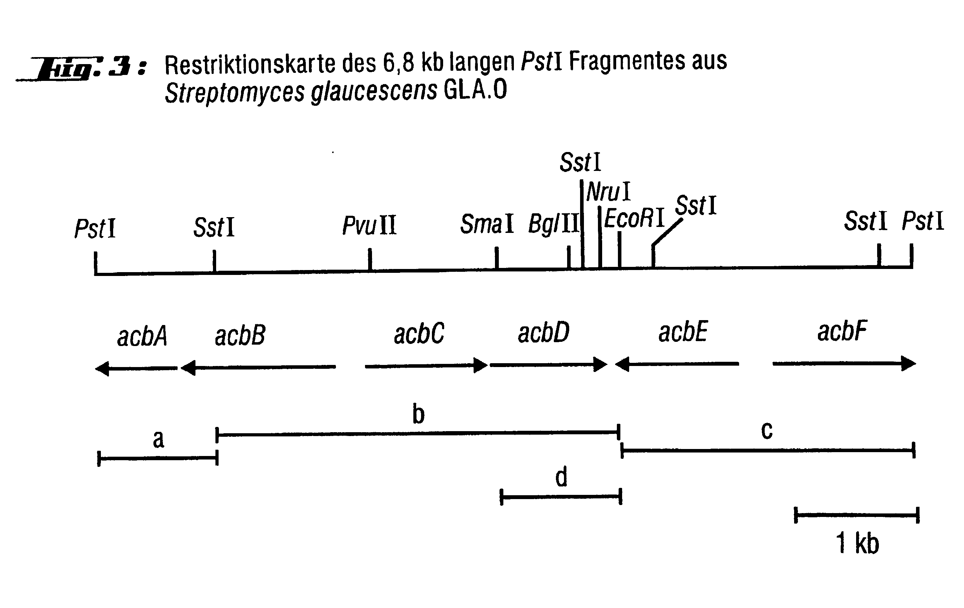

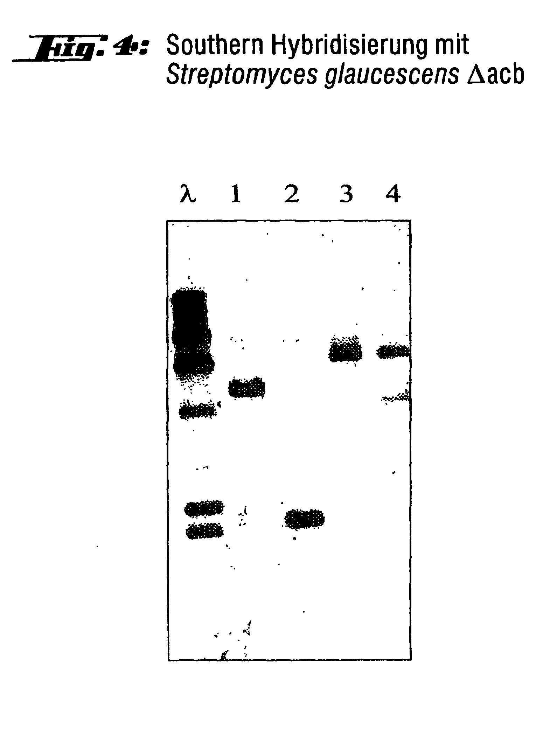

Die beiden Gene markieren unterschiedliche DNA-Bereiche (Abb. 1 und Abb. 2), wobei es sich bei den Fragmenten, die durch HstrE* markiert wurden, um Genfragmente aus der Hydroxystreptomycin-Biosynthese von Streptomyces glaucescens (DSM 40716) handeln muß. Die DNA-Sequenz ist nicht veröffentlicht, aber die hohe Homologie der von HstrE* abgleiteten Proteinsequenz zu StrE (Pissowotzki et al. (1991) Mol. Gen. Genet. 231: 113-123) aus der Streptomycinbiosynthese von Streptomyces griseus N2-3-11 (82% Identität) und die Übereinstimmung der durch HstrE* markierten DNA-Fragmente (Abb. 1) mit der publizierten Restriktionskarte des Hydroxystreptomycin Genclusters aus Streptomyces glaucescens (DSM 40716) (Retzlaff et al. (1993) Industrial Microorganisms. Basic and applied molecular genetics ASM, Washington DC, USA) läßt diesen Schluß zu. Die Fragmente, die durch die Sonde acbD* markiert wurden (Abb. 2), gehören einem bis dahin noch nicht untersuchten DNA Bereich zu. Dieser Bereich kodiert für die Biosynthese-Enzyme der Pseudo-Oligosaccharide aus Streptomyces glaucescens (DSM 40716).

### Beispiel 4: Klonierung des 6,8 kb langen Pstl Fragmentes

Unter anderem wird durch das PCR Fragment acbD* ein 6,8 kB langes Pstl DNA Fragment markiert (Abb. 2). Dieses DNA-Fragment wurde wie folgt isoliert. Der Bereich des Geles wurde mit einer Rasierklinge ausgeschnitten und die DNA mit einem Isolierungskit der Firma Pharmacia Biotech aus dem Gel isoliert und in das mit dem Restriktionsenzym Pstl geschnitte Plasmid pUC19 kloniert (Plasmid pacb1) und in den E. coli Stamm DH5α transformiert. Von diesen Platten wurden Einzelklone abgeimpft und eine Plasmid-DNA Isolierung mit diesen Klonen durchgeführt. Mit der DNA dieser Klone (250) wurde einer PCR Amplifikation mit den beschriebenen Primem 1 und 2 (Tab. 1) durchgeführt. Auf diese Weise wurde der entsprechende E. coli Klon, der das 6,8 kb lange Pstl Fragment enthält, isoliert.

### Beispiel 5: Sequenzierung des isolierten 6,8 kb langen Pstl DNA-Fragmentes

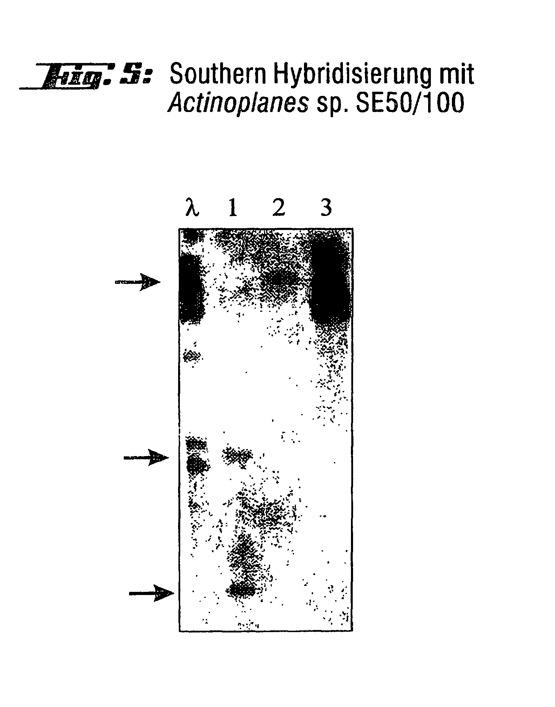

Die DNA wurde mit verschiedenen Restriktionsenzymen verdaut und einzelne DNA Fragmente in pUC19 kloniert. Anschließend wurde die DNA-Sequenz des gesamten Fragmentes bestimmt, welche in Tab. 4 (SEQ ID NO.: 7) gezeigt wird. Die DNA-Sequenz des 6,8 kb Pstl Fragmentes wurde nur teilweise durch ergänzende Sequenzierung des Gegenstranges bestätigt. Es wurden mehrere offene Leserahmen gefunden, die für verschiedene Proteine kodieren (Programme: CODONPREFERENCE, BlastX). Insgesamt konnten 6 kodierende Bereiche gefunden werden. Ein Gen mit hoher Homologie zu ATP-Bindeproteinen acbA, eine Aminotransferase acbB, eine dTDP-Glucose-Synthase acbC, eine dTDP-Glucose-Dehydratase acbD, ein Regulationsgen mit Homologien zur LacI Protein Familie acbE und ein Protein mit Ähnlichkeiten zu Zuckerbindenden-Proteinen acbF. Die Sequenz der Gene acbA und acbF wurde nur teilweise bestimmt. Die Homologien zu anderen Proteinen aus den Datenbanken und die Eigenschaften der putativen Proteine sind in Tab. 3 zusammengefaßt. Abb. 3 zeigt zusammenfassend eine Restriktionskarte des Fragmentes mit den wichtigsten im Text erwähnten Restriktionsschnittstellen und die Anordnung der identifizierenden offenen Leserahmen.

**Tabelle 3 : Analyse der identifzierten offenen Leserahmen auf dem 6,8 kb langen Pstl Fragment aus Streptomyces glaucescens (DSM 40716)**

| ORF | Aminosäuren | MG | FastA^{§} | Identität% | Accession Nummer ^{§} |
|---|---|---|---|---|---|
| acbA | 239 | * | MalK, E coli | 29 % | P02914 |
| acbB | 429 | 45618 | DgdA, Burkholderia cepacia | 32 % | P16932 |
| acbC | 355 | 37552 | StrD, Streptomyces griseus | 60 % | P08075 |
| acbD | 325 | 35341 | StrE, Streptomyces griseus | 62 % | P29782 |
| acbE | 345 | 36549 | DegA, Bacillus. subtilis | 31 % | P37947 |
| acbF | 396 | * | MalE, E. coli | 22 % | P02928 |

| | | | | | |
|---|---|---|---|---|---|
| * unvollständiger offener Leserahmen; ^{§} Swiss-Prot Datenbank (Release 32) | | | | | |

### Beispiel 6: Deletion der Gene für die Pseudo-Oligosaccharid-Biosynthese acbBCD vom Chromosom von Streptomyces glaucescens (DSM 40716)

Ein Beweis, daß das identifizierte DNA-Fragment für Gene der Pseudo-Oligosaccharid-Biosynthese kodiert, wurde wie folgt geführt. Ein Genbereich von 3,4 kb (EcoRI-SstI Fragment b, Abb. 3) wurde durch das Erythromycinresistenzgen (1,6 kb) ersetzt und mit flankierenden DNA-Bereichen aus dem 6,8 kb Pstl-Fragmentes (pacb1) in das temperatursensitive Plasmid pGM160 kloniert. Die Konstruktion des Plasmides erfolgte, wie im Anschluß beschrieben: Aus dem Plasmid pacb1 wurde das 2,2 kb lange EcoRI-HindIII Fragment (c, Abb.3) in pGEM7zf (Promega, Madison, WI, USA; Plasmid pacb2), sowie das 1 kb lange Sstl Fragment aus pacb1 (a, Abb. 3) in pUC19 kloniert (Plasmid pacb3). Anschließend wurde eine Ligation mit folgenden Fragmenten durchgeführt. Das Plasmid pGM160 (Muth et al. (1989) Mol. Gen Genet. 219:341-348) wurde mit BamHI/HindIII geschnitten, das Plasmid pacb2 mit XbaI/BamHI (c, Abb. 3), das Plasmid pacb3 mit EcoRI/HindIII (a, Abb. 3) und das Plasmid pIJ4026 (Bibb et al. (1985) Gene 38:215-226) zur Isolierung des 1,6 kb langen ermE Resistenz Genes mit EcoRl/Xbal. Die Fragmente wurden in einem Ansatz ligiert und in E. coli DH5α transformiert und auf Ampicillin selektioniert. Das resultierende Plasmid pacb4 wurde aus E. coli DH5α isoliert, mittels Restriktionsverdau auf seine Richtigkeit überprüft und anschließend in S. glaucescens (DSM 40716) durch Protoplasten Transformation übertragen. Die Transformanten wurden mit Thiostrepton bei 27°C auf R2YENG Agar selektioniert. Anschließend wurde eine Inkubation der Transformanden bei der nicht-permissiven Temperatur von 39°C durchgeführt und somit die Integration des Plasmides in das Genom über homologe Rekombination eingeleitet (Selektion mit Thiostrepton (25µg/ml) und Erythromycin (50 µg/ml)). Nur solche Klone können unter diesen Bedingungen wachsen, bei denen das Plasmid in das Genom integriert ist. Entsprechende Klone wurden isoliert, zur Sporulation gebracht (Medium 1, s.u.) und auf Erythromycin-haltigem Agar (Medium 1) ausplattiert. Von dieser Platte wurden wieder Einzelklone isoliert und sowohl auf Thiostrepton- als auch Erythromycinhaltigen Medien ausgestrichen. Solche Klone wurden analysiert, die Erythromycin-resistent aber nicht mehr Thiostrepton-resistent waren. Bei diesen Klonen waren die Gene acbBCD durch das ermE ersetzt worden. Es wurden mehrere Klone untersucht und schließlich der Stamm S. glaucescens (DSM 40716) Δacb als Referenzstamm (Erythromycin-resistent, Thiostrepton-sensitiv) zur weiteren Untersuchung ausgewählt.

### Medium 1

| | |
|---|---|
| Hefeextrakt | 4 g/L |
| Malzextrakt | 10 g/L |
| Glucose | 4 g/L |
| Agar | 15 g/L |
| pH | 7,2 |

In einem weiteren Experiment wurde überprüft, ob der entsprechende Stamm noch Acarbose produzierte. Einige Klone wurden angezogen und im Bioassay auf die Bildung des α-Amylase Hemmstoffes untersucht, aber keine Aktivität gefunden. Die Mutanten wurden anschließend durch Southern-Hybridisierung weiter charakterisiert. Die Integration des ermE Genes hatte an der vorhergesagten Stelle stattgefunden. Abb. 4 zeigt eine Southern-Hybridisierung, die mit dem Wildtyp und der Deleletionsmutante Streptomyces glaucescens (DSM 40716) Δacb durchgeführt wurde. Als Sonde wurde das Sstl Fragment von pacb3 verwendet. Die chromsomale DNA wurde aus dem Wildtyp und der Mutanten isoliert und mit den Enzymen Pstl und PstI/HindIII verdaut. Das Fragmentmuster der Deletionsmutanten entspricht dem vorhergesagten Rekombinationsereignis. Der Wildtyp weist das unveränderte 6,8 kb lange Pstl Fragment auf, während die Mutante ein um 1,8 kb verkürztes Fragment aufweist (vergleiche Spur 1 und 3, Abb. 4). Durch die Integration des ermE Resistenzgenes wurde außerdem eine interne Hindlll-Schnittstelle in das Pstl Fragment eingeführt (vergleiche Spur 2 und 4, Abb. 4)

### Beispiel 7: Hemmung von α-Amylase durch Acarbose

Es konnte durch einen enzymatischen Test zum Stärke-Nachweis (TC-Starch, Boehringer-Mannheim, Cat. No. 297748) gezeigt werden, daß die aus Streptomyces glaucescens (DSM 40716) isolierte Verbindung α-Amylase hemmt. Testprinzip: Stärke wird durch Amyloglucosidase in D-Glucose gespalten. Die Glucose wird anschließend durch Hexokinase zu Glucose-6-phosphat und diese durch Glucose-6-phosphat-dehydrogenase zu D-gluconate-6-phosphat umgesetzt. Hierbei entsteht NADPH, dessen Bildung photometrisch bestimmt werden kann. Acarbose hemmt die α-Amylase und verhindert damit die Bildung von D-Glucose und letztendlich auch die Bildung von NADPH.

### Beispiel 8: Medium zur Anzucht von S. glaucescens (DSM 40716) und Produktion von Acarbose

Die Fermentation wurde in 500 ml Erlenmeyerkolben mit einem seitlichen Einstich und 100 ml Medium 2 bei 27°C auf einer rotieren Schüttelmaschine bei 120 upm durchgeführt. Die Fermentation wurde nach 2 oder 3 Tagen abgebrochen. Die Pseudo-Oligosaccharide konnten im Plattendiffusionstest gemäß Beispiel 9 nachgewiesen werden. Mit Medium 3 wurden keine α-Amylase-Hemmstoffe produziert. Das bedeutet, daß die Produktion der Pseudo-Oligosaccharide durch Glucose gehemmt wird. Auch andere Zucker wie Maltose, Rohrzucker oder komplexe Zuckerquellen (Malzextrakt) können für die Produktion von Pseudo-Oligosacchariden mit S. glaucescens (DSM 40716) in Frage kommen.

### Medium 2:

| | |
|---|---|
| Sojamehl | 20 g/L |
| Stärke | 20 g/L |
| pH | 7,2 |

### Medium 3:

| | |
|---|---|
| Sojamehl | 20 g/L |
| Glucose | 20 g/L |
| pH | 7,2 |

### Beispiel 9: Biotest unter Verwendung von Mucor miehei

Eine Sporensuspension des Stammes Mucor miehei wurde in Agar (Medium 5) eingegossen (10⁵ Sporen/ml) und von diesem Ansatz jeweils 10 ml in Petrischalen gegossen. Papiertestplättchen (Durchmesser 6 mm) wurden mit 10 *µ*l Acarbose- (1 mg/ml) oder mit einer Probe aus einer S. glaucescens Kultur beschickt und auf die Testplatten aufgelegt. Die Inkubation der Platten erfolgte bei 37°C. Es zeigten sich Hemmhöfe auf dem Stärke-haltigem Medium 5. Als Kontrolle diente eine Platte, die mit Glucose (Medium 4) statt mit Stärke angesetzt wurde. Auf diesem Medium bildete sich kein Hemmhof um die mit Wirkstoff beladenen Filterplättchen.

### Medium 4 und 5:

| | |
|---|---|
| KH₂PO₄ x 3 H₂O | 0,5 g |
| MgSO₄ x 7 H₂O | 0,2 g |
| NaCl | 0,1 g |
| Ammoniumsulfat | 5 g |
| Yeast nitrogen base | 1,7 g |
| Glucose (4) oder Stärke (5) | 5 g |
| Agar | 15 g |

### Beispiel 10: Transformation S. glaucescens (DSM 40716).

Protoplasten des Stammes Streptomyces glaucescens wurden wie bei Motamedi and Hutchinson ((1987) PNAS USA 84: 4445-4449) beschrieben isoliert und die isolierte Plasmid-DNA mittels PEG-Transformation wie bei Hopwood et al. ((1985) Genetic manipulations of Streptomyces: a laboratory manual. The John innes Foundation, Norwich UK) erläutert in die Zellen übertragen. Die Regeneration der Protoplasten wurde auf R2YENG Medium bei 30°C durchgeführt (Motamedi and Hutchinson (1987) PNAS USA 84: 4445-4449). Die Agarplatten wurden nach 18 h mit einer thiostreptonhaltigen Lösung überschichet und bei 30°C inkubiert (Endkonzentration Thiostrepton: 20 *µ*g/ml).

### Beispiel 11: Isolierung der Pseudo-Oligosaccharide aus Streptomyces glaucescens , (DSM 40716) HPLC-Analyse und Massenspektroskopie

### Isolierung

Die Kulturbrühe wurde durch Filtration vom Mycel getrennt. Das so gewonnen Kulturfiltrat wird anschließend auf eine XAD16 Säule aufgetragen, diese mit Wasser gewaschen und die aktiven Komponenten mit 30% Methanol eluiert. Das Eluat wurde bis zur wässrigen Phase eingeengt und mit Essigester extrahiert, um lipophile Verunreinigungen zu entfernen. Die wässrige Phase wurde anschließend eingeengt und die aktiven Komponenten mit einer Biogel P2 Säule in 5% Methanol weiter gereinigt. Die einzelnen Fraktionen werden mit einem Fraktionssammler aufgefangen. Die Analyse der einzelnen Fraktionen erfolgte mittels Biotest mit Mucor miehei. Aktive Eluate wurden zur weiteren Reinigung auf Biogel P2 in 5% Methanol rechromatographiert. Das so isolierte Material wurde mittels HPLC und MS weiter untersucht.

### HPLC

Säule: Nucleosil^{®} 100 C-18
Laufmittel 0,1 % Phosphorsäure = A/Acetonitril = B
Gradient: von 0-100% B in 15 min
Detektion: 215 nm
Fluß 2 ml/min
Injektionsvolumen: 10-20 µl

Die Pseudo-Oligosaccharid-Präparation aus S. glaucescens (DSM 40716) konnte mittels HPLC nicht von der authentischen Acarbose unterschieden werden. Sowohl die Retentionszeit als auch das UV-Absorbtionsspektrum der beiden Komponenten waren in diesem Laufmittelsystem identisch. Das Pseudo-Oligosaccharid-Gemisch wurde unter diesen Bedingungen nicht aufgetrennt.

### Massenspektroskopische Analyse (MS)

Die Bestimmung der Molekulargewichte und des Fragmentierungsmusters authentischer Acarbose und der aus Streptomyces glaucescens (DSM 40716) isolierten Pseudo-Oligisaccharide erfolgte mittels Electro-Spray MS. Die Analyse von kommerziell erhältlicher Acarbose von Bayer (Glucobay) ergab einen Massenpeak bei 645,5 (Acarbose). Die aufgereinigten Proben aus S. glaucescens (DSM 40716) enthalten eine Mischung verschiedener Pseudo-Oligosaccharide mit unterschiedlicher Länge in der Zuckerseitenkette: 969 (Acarbose + 2 Glucoseeinheiten), 807 (Acarbose + 1 Glucoseeinheit), 645 (entspricht der authentischen Acarbose). Bei der Fragmentierung der Acarbose und der aus S. glaucescens (DSM 40716) isolierten Verbindung mit dem Molekulargewicht 645 entstehen die gleichen Molekülbruchstücke: 145 (4-Amino-4,6 deoxyglucose) 303 (Acarviosin) und 465 (303 mit einer Glucoseeinheit).

Auch Actinoplanes sp. SE50 produziert eine Mischung von Acarbosemolekülen mit unterschiedlichen langen Zuckerseitenketten (Truscheit (1984) VIIIth International Symposium on Medicinal Chemistry, Proc. Vol. I. Swedish Academy of Pharmaceutical Sciences, Stockholm, Sweden). Die Länge der Zuckerseitenketten kann durch die Wahl der Fermentationsparameter und des Substrates in der Nährlösung beinflußt werden.

### Beispiel 12: Southern-Hybridisierung mit Actinoplanes sp. SE50/110 (ATCC31044)

Aus dem Stamm Actinoplanes sp. SE50/100 wurde die chromosomale DNA isoliert und mit Restriktionsenzymen verdaut (Pstl und BamHI). Anschließend wurde eine Southern-Hybridisierung mit einer Sonde, die den kodierenden Bereich der dTDP-glucose 4, 6 dehydratase acbD aus Streptomyces glaucescens (DSM 40716) umfasst (Fragment d, Abb. 3), durchgeführt. Die Sonde hybridisiert mit distinkten Banden aus Actinoplanes sp. SE50/110 (Abb. 5, Spur 1 und 2). Damit ist die Möglichkeit der Isolierung der entsprechenden Fragmente aus Actinoplanes sp. SE50/100 und weiteren Stammlinien gegeben. Ob diese DNA-Bereiche tatsächlich an der Biosynthese der Acarbose beteiligt sind, muß noch in weiteren Untersuchungen gezeigt werden. Alternativ könnten auch die PCR Primer 1 und 2 (Tab. 1) zur Amplifikation der dTDP-Glucose 4, 6 Dehydratase aus Actinoplanes sp. verwendet werden.

### Legenden:

- Abb. 1:: Southern Hybridisierung mit S. glaucescens (DSM 40716). Spur 1: PstI, Spur 2: BamHI, Spur 3: BglII. Als Sonde wurde das markierte PCR-Fragment HstrE* verwendet. Markierung von DNA-Fragmenten, die an der Biosynthese von Hydroxystreptomycin beteiligt sind.
- Abb. 2:: Southern Hybridisierung mit S. glaucescens (DSM 40716). Spur 1: PstI, Spur 2: BamHI, Spur 3: BglII. Als Sonde wurde das markierte PCR-Fragment acbD* verwendet. Markierung von DNA-Fragmenten, die an der Biosynthese der Pseudo-Oligosaccharide beteiligt sind.
- Abb. 3:: Restriktionskarte des 6,8 kb langen PstI Fragmentes aus Streptomyces glaucescens (DSM 40716). Offene Leserahmen und deren jeweilige Transkriptionsrichtung sind durch Pfeile angedeutet. Die Fragmente a, b, c und d kennzeichnen DNA-Bereiche, die im Text näher erläutert werden.
- Abb. 4:: Southern Hybridisierung mit Streptomyces glaucescens Δacb: Spur 1: PstI, Spur 2: PstI/HindIII, und Streptomyces glaucescens (DSM 40716) Spur 3: PstI, Spur: 4: PstI/HindIII. Als Sonde wurde das markierte 1,0 kb lange SstI-Fragment a (Abb. 3) verwendet.
- Abb. 5:: Southern Hybridisierung mit Actinoplanes sp. SE50/100: Spur 1: PstI, Spur 2: BamHI und Streptomyces glaucescens (DSM 40716) Spur 3: Pstl. Als Sonde wurde das markierte 1,0 kb lange SmaI/EcoRI Fragment d (dTDP-glucose-4,6-dehydratse, Abb. 3) verwendet. Die Pfeile weisen auf die markierten DNA Fragmente hin (BamHI: 2.1 und 0,7 kb, Pstl: ~11-12 kb)
- Tab. 4:: DNA-Sequenz des 6,8 kb langen PstI Fragmentes aus Streptomyces glaucescens (DSM 40716) (SEO ID NO.: 7). Die abgeleitete Aminosäuresequenz (SEQ ID NO.: 8-13) der identifizierten offenen
Leserahmen ist unter der DNA-Sequenz angegeben. Start-, Stopcodons und potentielle Ribosomen-Bindungsstellen sind unterstrichen.
acbA: SEQ ID NO.: 8
acbB: SEQ ID NO.: 9
acbC: SEQ ID NO.: 10
acbD: SEC ID NO.: 11
acbE: SEQ ID NO.: 12
acbF: SEQ ID NO.: 13

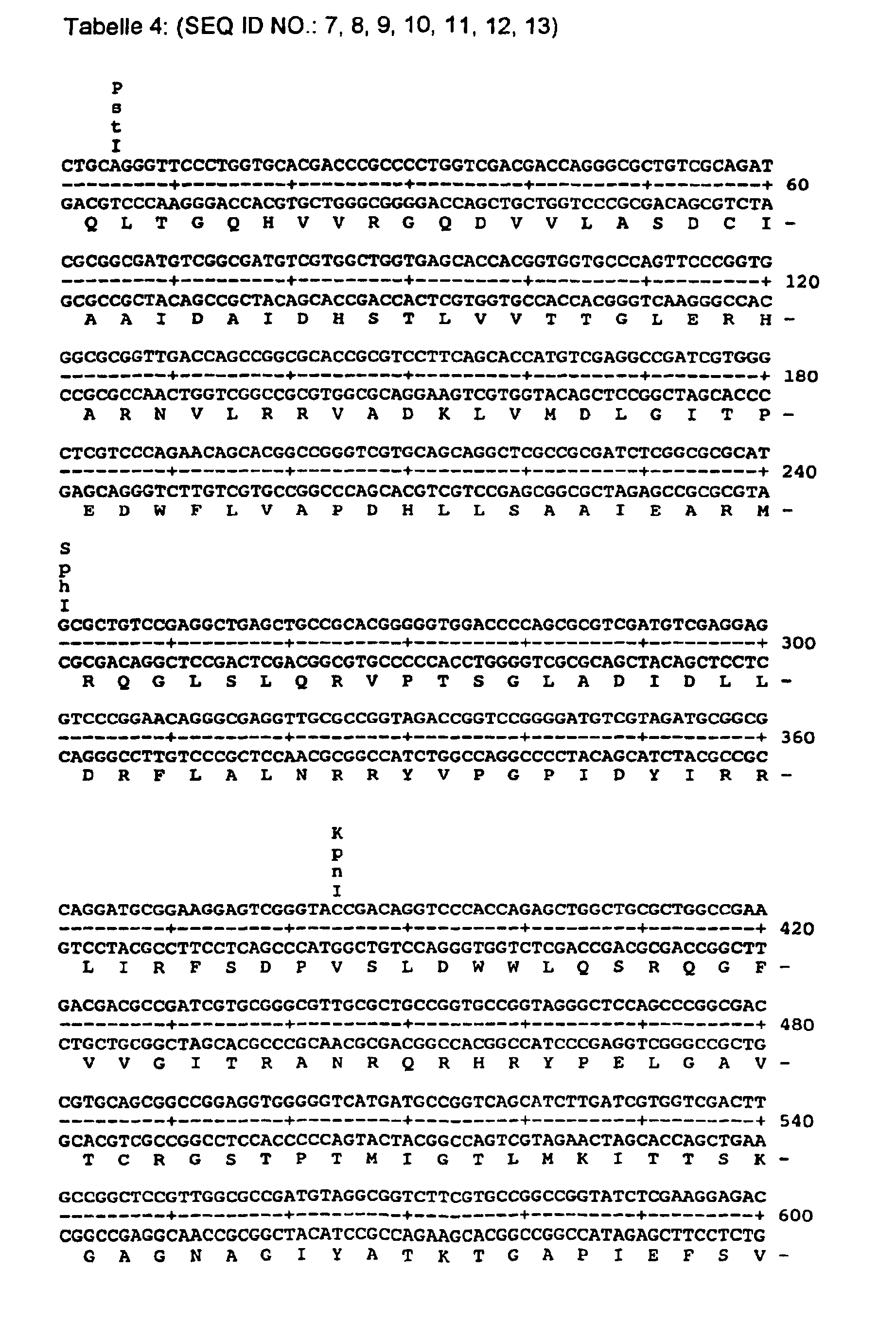

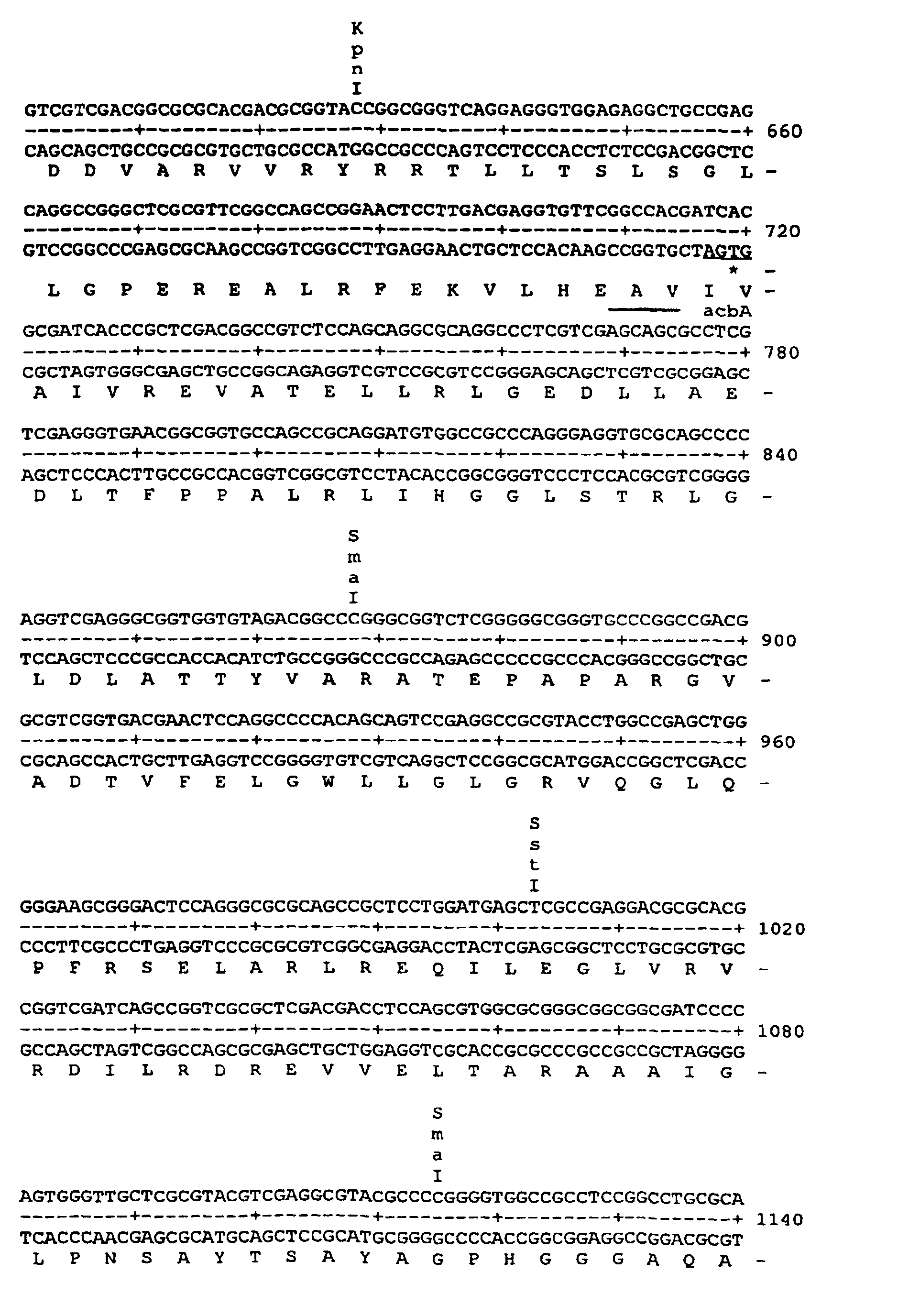

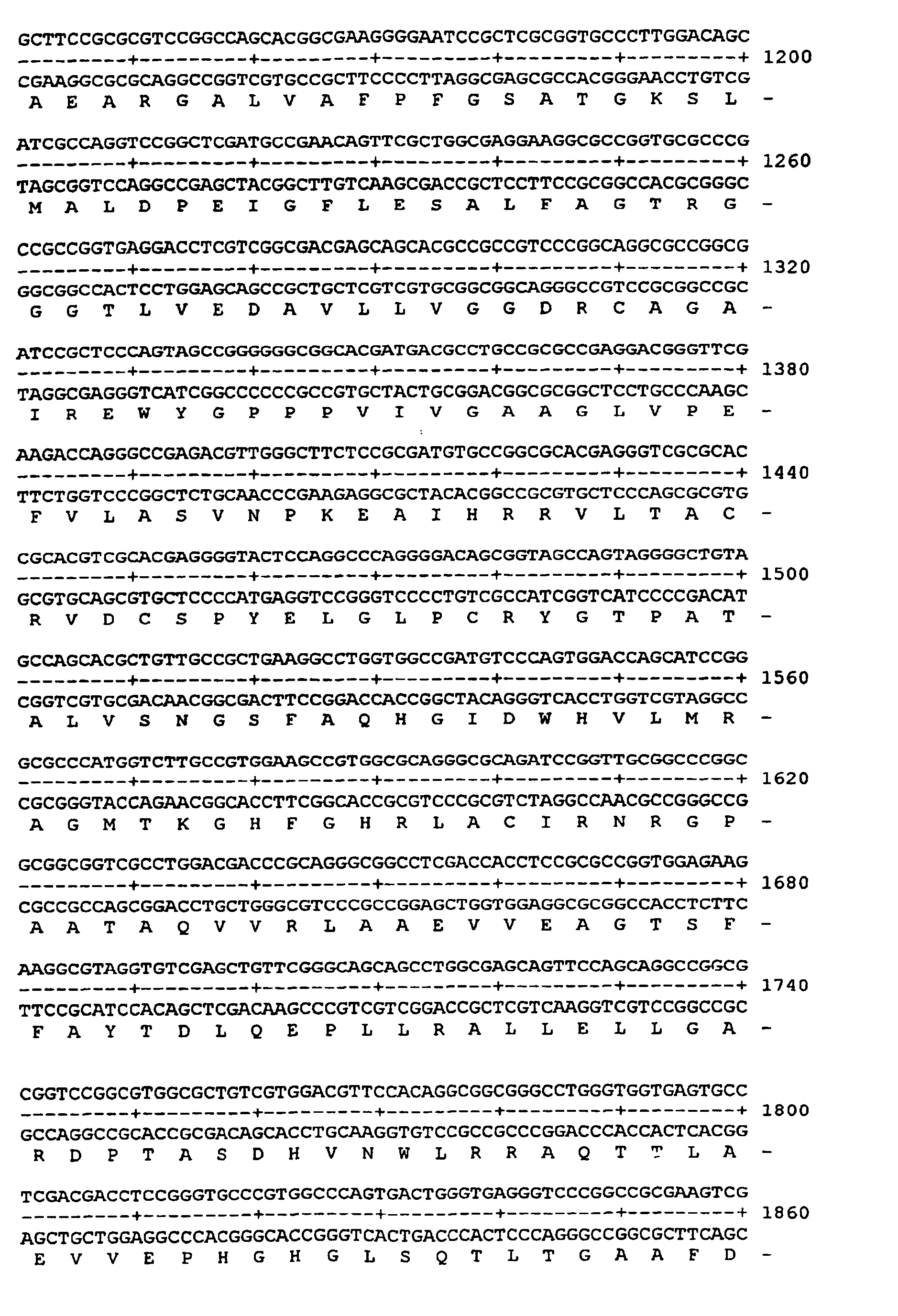

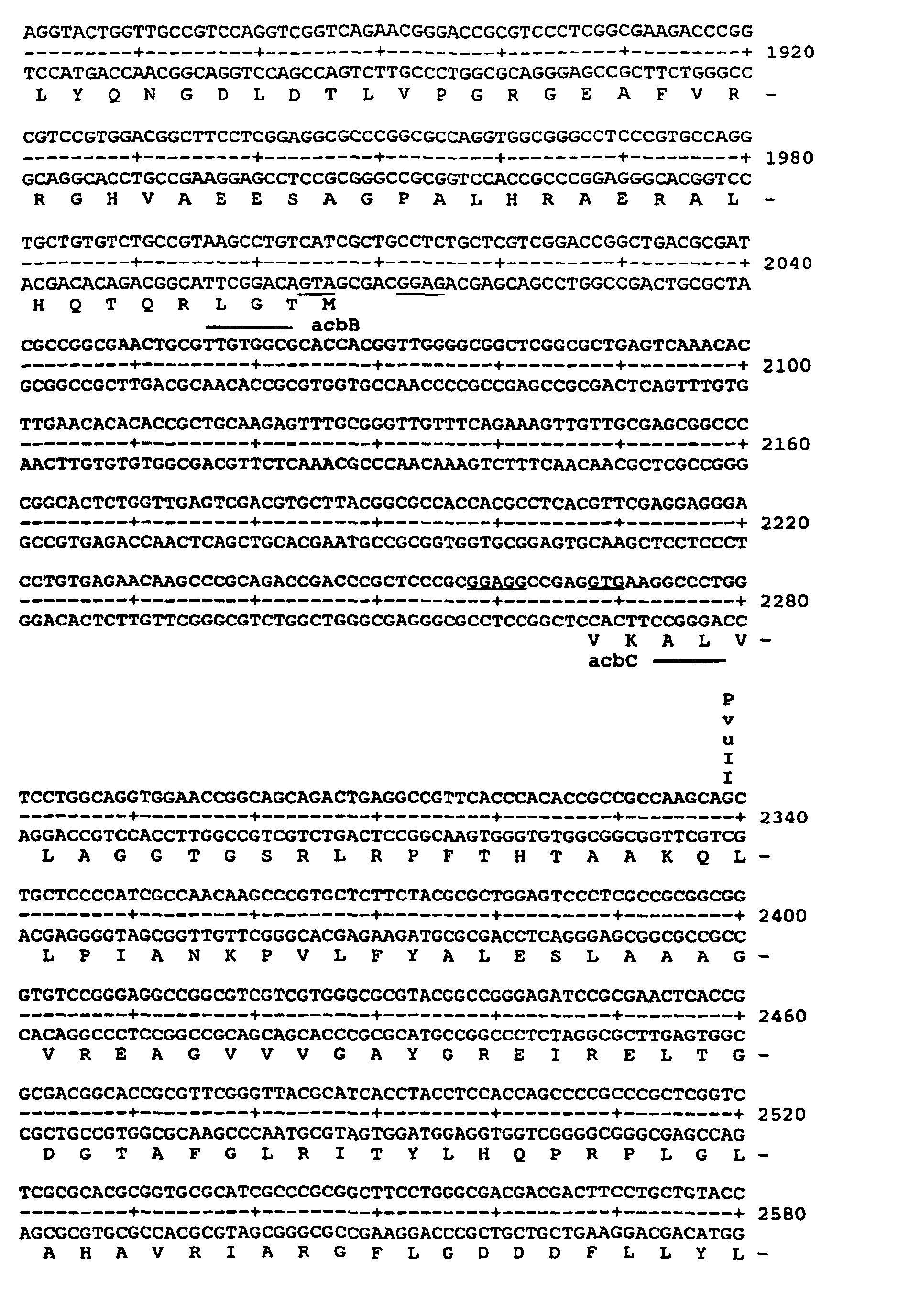

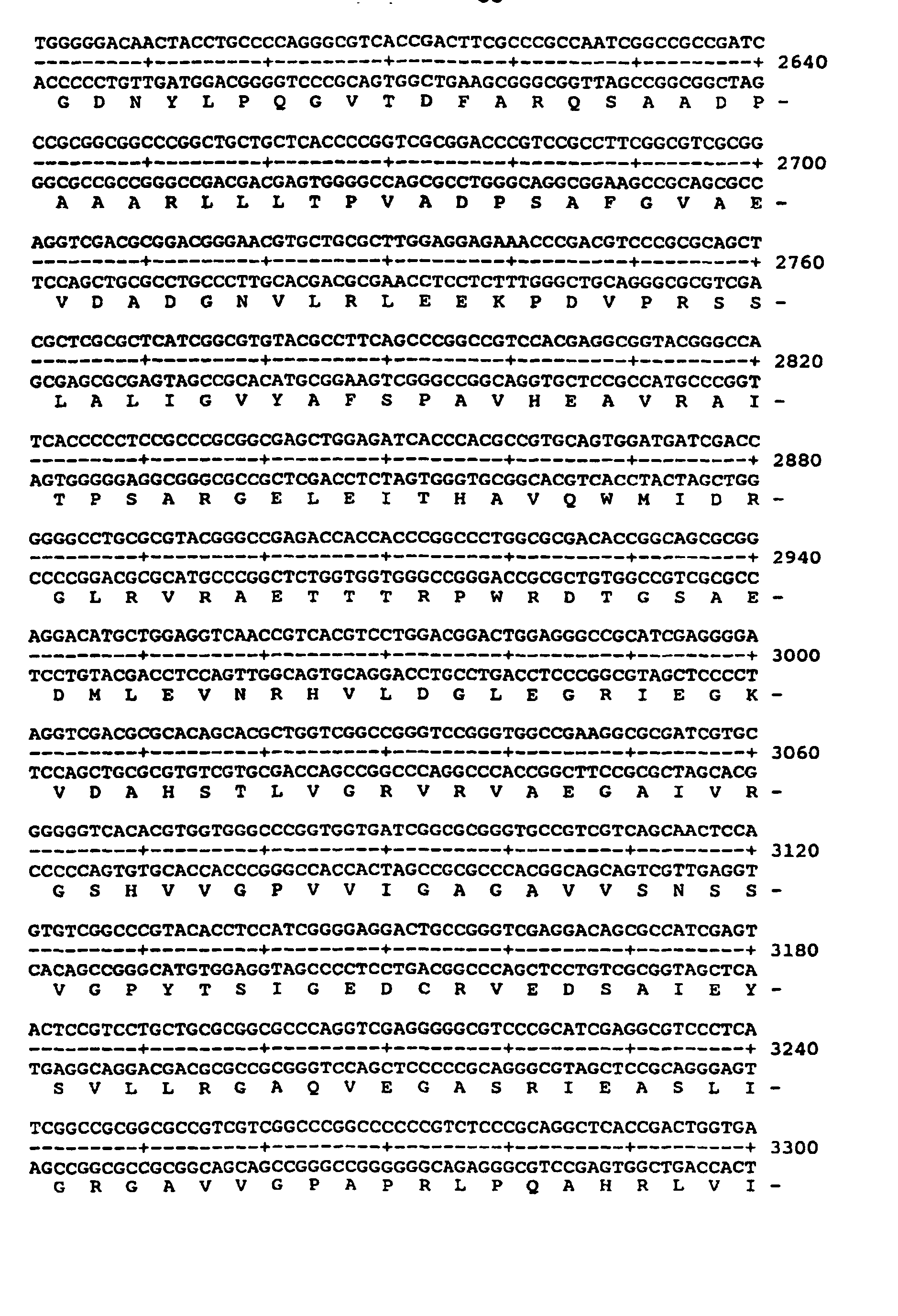

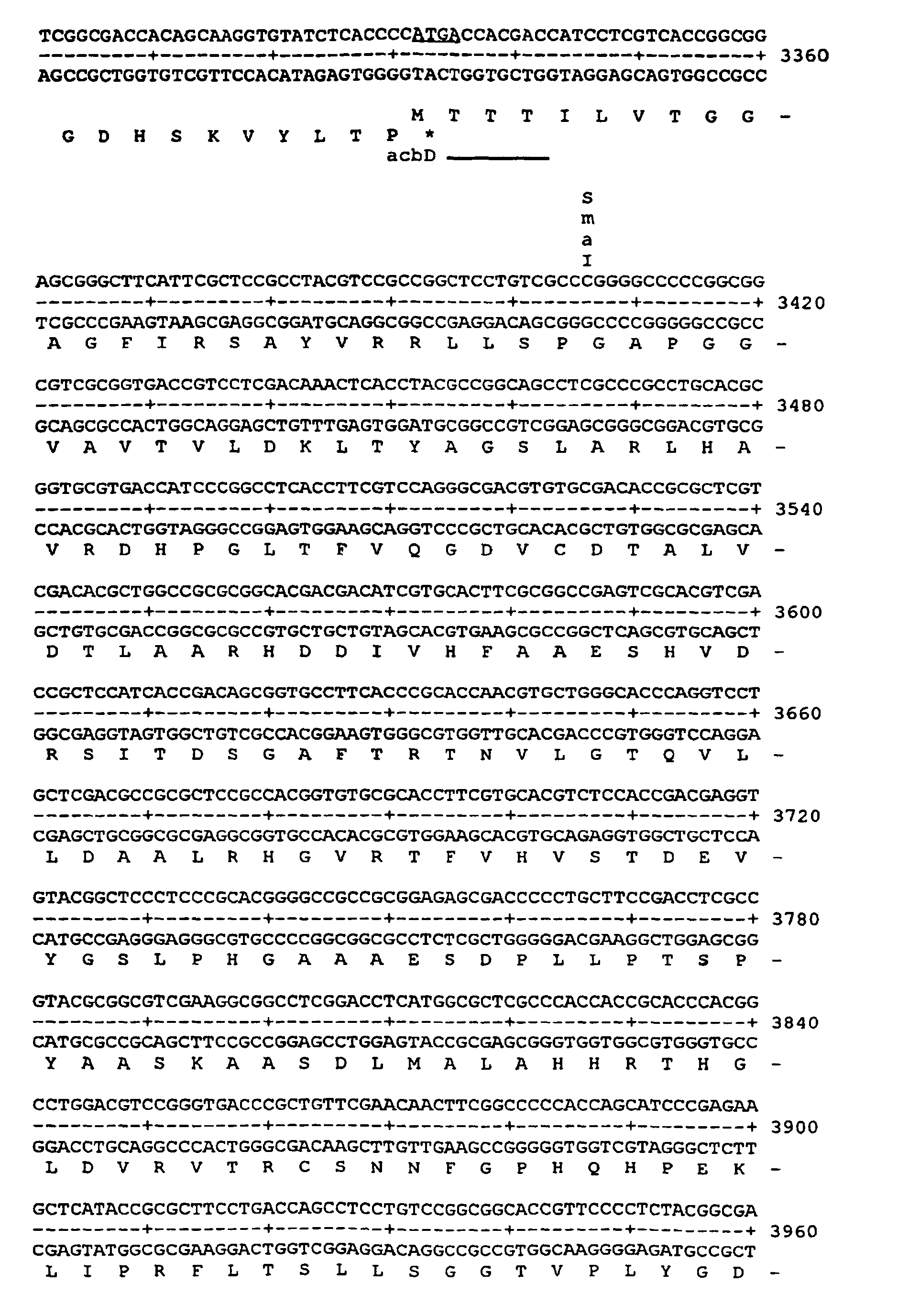

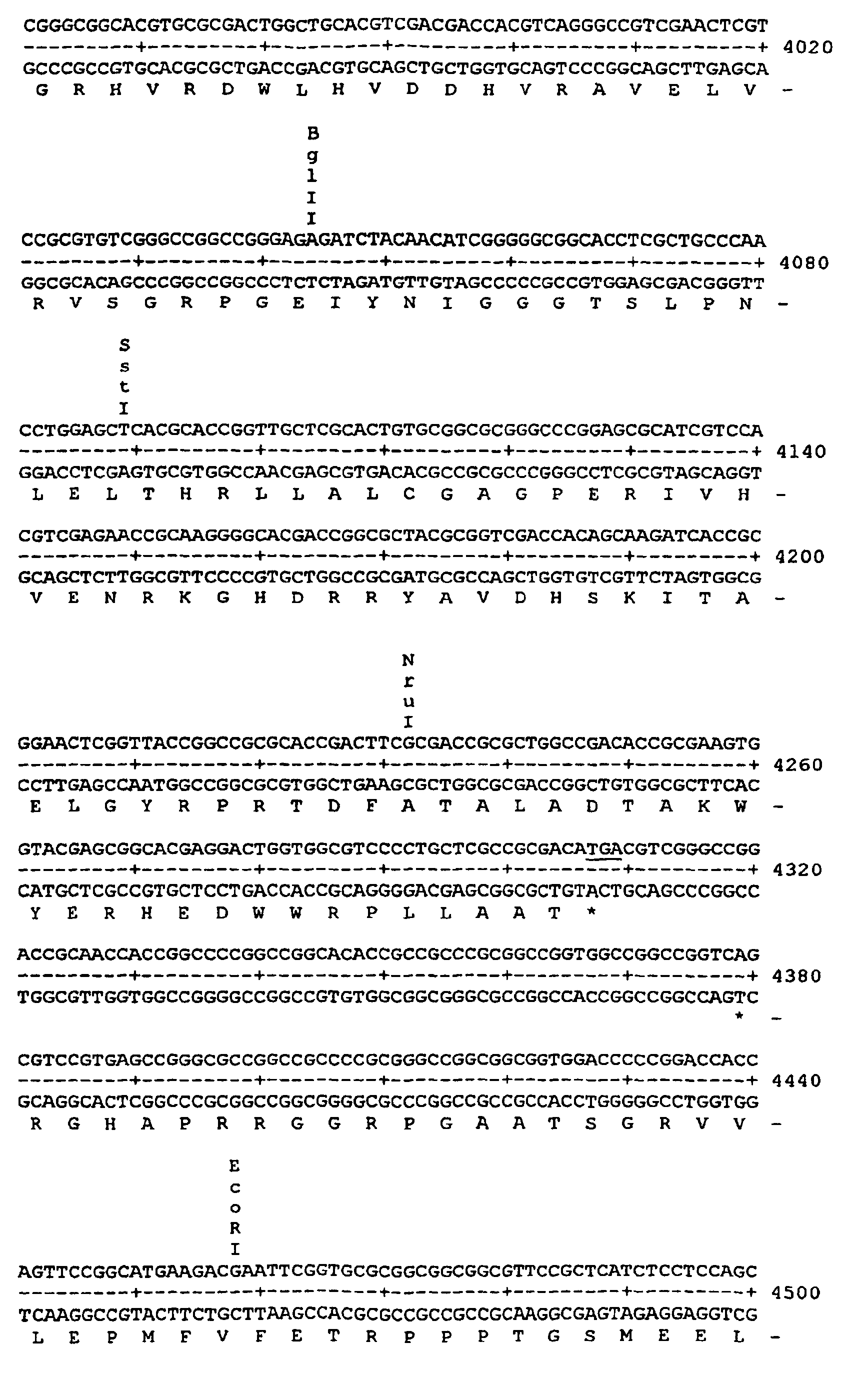

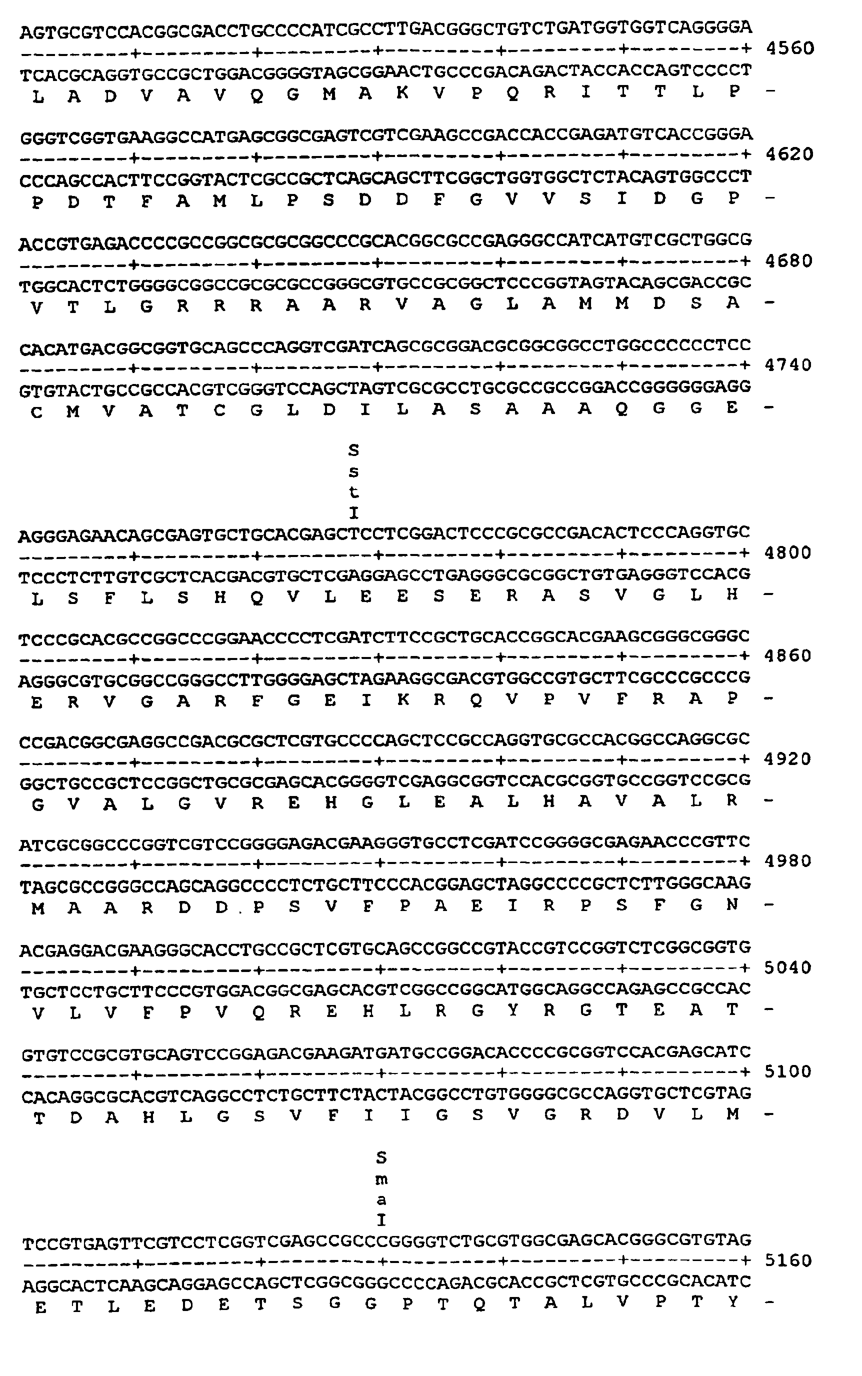

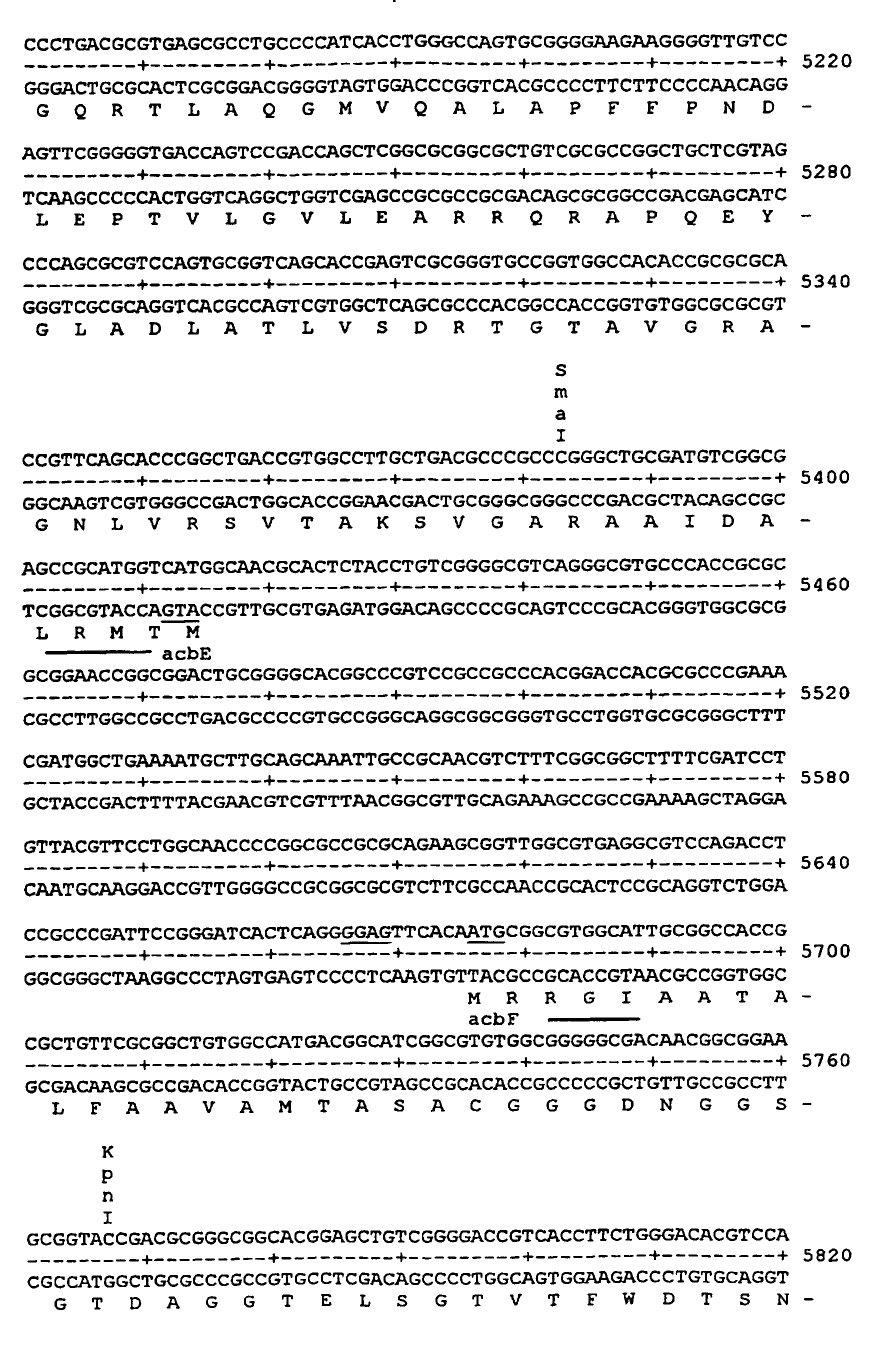

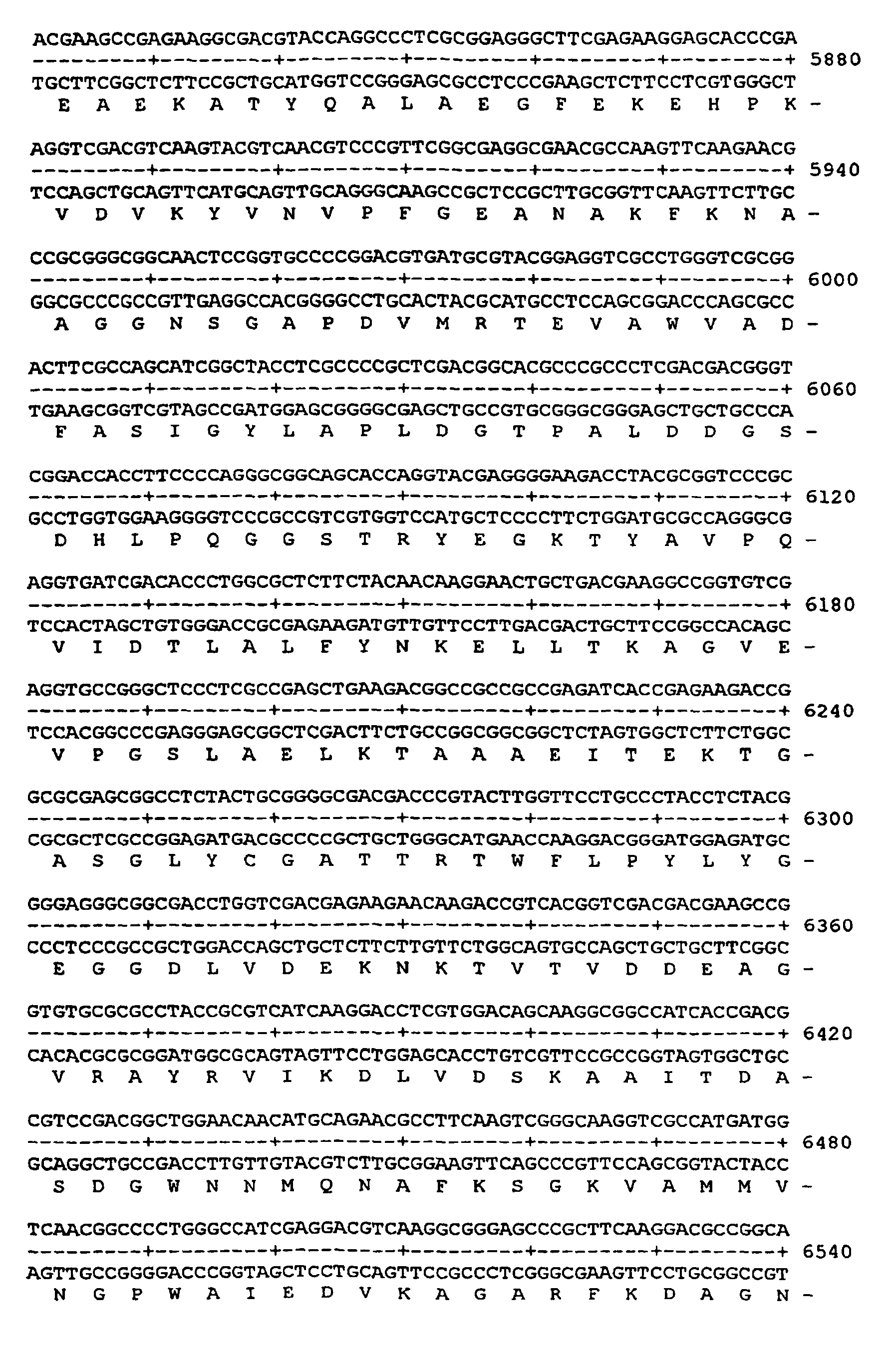

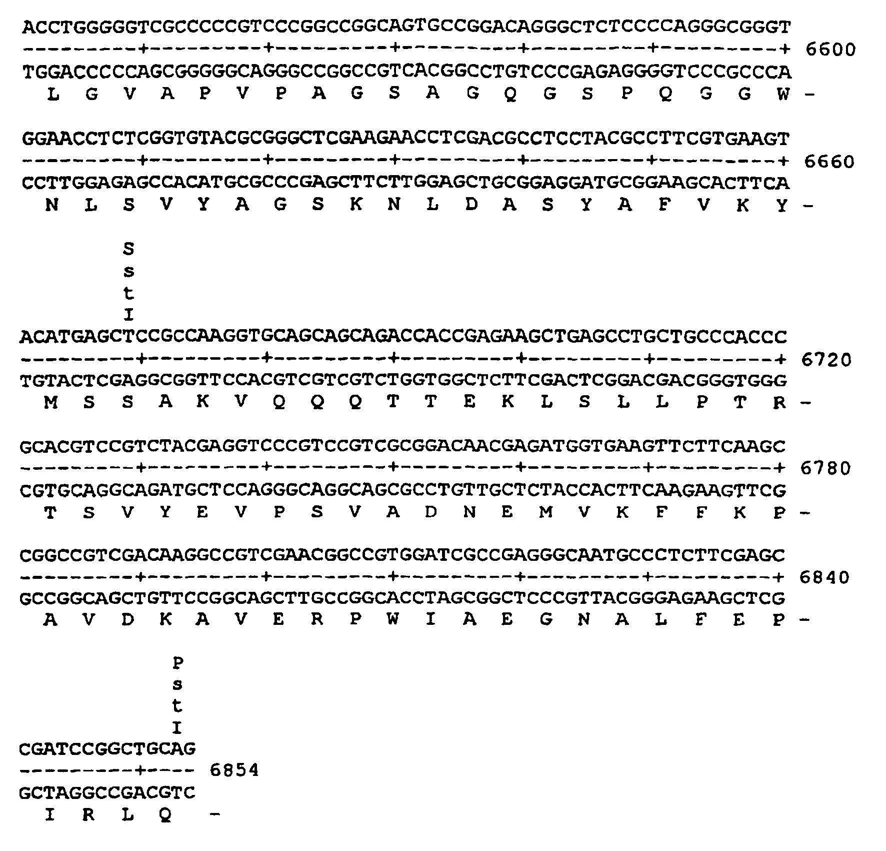

## Patentansprüche

1. DNA-Molekül, enthaltend die Gene für die Biosynthese von Acarbose, **gekennzeichnet**, daß
(a) es eine DNA-Sequenz gemäß Tabelle 4 enthält; oder
(b) es eine DNA-Sequenz enthält, die in der Lage ist, mit dem DNA-Molekül gemäß (a) unter stringenten Bedingungen zu hybridisieren oder
(c) es eine DNA-Sequenz enthält, die aufgrund der Degeneriertheit des genetischen Codes von den DNA-Molekülen gemäß (a) und (b) verschieden ist, jedoch die Expression der entsprechend mit den DNA-Molekülen gemäß (a) und (b) exprimierbaren Proteinen ermöglicht.

2. DNA-Molekül gemäß Anspruch 1, **dadurch gekennzeichnet, daß** es als die unter (a) genannte Sequenz die DNA-Sequenz der Nukleotide 1 bis 720 (acbA-Gen) nach Tabelle 4 enthält.

3. DNA-Molekül gemäß Anspruch 1, **dadurch gekennzeichnet, daß** es als die unter (a) genannte Sequenz die DNA-Sequenz der Nukleotide 720 bis 2006 (acbB-Gen) nach Tabelle 4 enthält.

4. DNA-Molekül gemäß Anspruch1, **dadurch gekennzeichnet, daß** es als die unter (a) genannte Sequenz die DNA-Sequenz der Nukleotide 2268 bis 3332 (acbC-Gen) nach Tabelle 4 enthält.

5. DNA-Molekül gemäß Anspruch 1, **dadurch gekennzeichnet, daß** es als die unter (a) genannte Sequenz die DNA-Sequenz der Nukleotide 3332 bis 4306 (acbD-Gen) nach Tabelle 4 enthält.

6. DNA-Molekül gemäß Anspruch 1, **dadurch gekennzeichnet, daß** es als die unter (a) genannte Sequenz die DNA-Sequenz der Nukleotide 4380 bis 5414 (acbE-Gen) nach Tabelle 4 enthält.

7. DNA-Molekül gemäß Anspruch 1, **dadurch gekennzeichnet, daß** es als die unter (a) genannte Sequenz die DNA-Sequenz der Nukleotide 5676 bis 6854 (acbF-Gen) nach Tabelle 4 enthält.

8. Oligonukleotid-Primer zur PCR-Aplififzierung des DNA-Moleküls gemäß Anspruch 1.

9. Vektor, enthaltend ein DNA-Molekül gemäß einem oder mehreren der Ansprüche 1 bis 8.

10. Vektor gemäß Anspruch 9 zur Verwendung in einem Verfahren zur Ausschaltung oder Veränderung von natürlichen Acarbose-Biosynthesegenen in einem Acarbose-produzierenden Mikroorganismus.

11. Vektor gemäß Anspruch 10, **dadurch gekennzeichnet, dass** er ausgewählt wird aus der Gruppe enthaltend pGM160 oder verwandte Vektoren.

12. Vektor gemäß Anspruch 9, **dadurch gekennzeichnet, daß** es ein Expressionsvektor ist und das besagte DNA-Molekül operativ mit einer Promotersequenz verbunden ist.

13. Vektor gemäß Anspruch 12, geeignet zur Expression in Wirtsorganismen, ausgewählt aus der Gruppe bestehend aus E. coli, Bacillus subtilis, Streptomyces-, Actinoplanes-, Ampullariella-, Streptosporangium-Stämmen, Streptomyces hygroscopicus var. limoneus, Streptomyces glaucescens, sowie Aspergillus niger, Penicillium chrysogenum und Saccharomyces cerevisiae ausgewählt wird.

14. Vektor gemäß Anspruch 12, geeignet zur Expression in Streptomyces glaucescens (DSM 40716) oder Actinoplanes sp.

15. Wirtszelle, transformiert mit einem Vektor gemäß einem der Ansprüche 9 bis 14.

16. Wirtszelle gemäß Anspruch 15, **dadurch gekennzeichnet, daß** sie aus der Gruppe bestehend aus E. coli, Bacillus subtilis, Streptomyces-, Actinoplanes-, Ampullariella-, Streptosporangium-Stämmen, Streptomyces hygroscopicus var. limoneus, Streptomyces glaucescens, sowie Aspergillus niger, Penicillium chrysogenum und Saccharomyces cerevisiae ausgewählt wird.

17. Wirtszelle gemäß Anspruch 16, **dadurch gekennzeichnet, daß** sie aus der Gruppe bestehend aus Streptomyces glaucescens (DSM 40716) und Actinoplanes sp. ausgewählt wird.

18. Protein (acbA-Genprodukt), kodiert von einer DNA gemäß Anspruch 2.

19. Protein (acbB-Genprodukt), kodiert von einer DNA gemäß Anspruch 3.

20. Protein (acbC-Genprodukt), kodiert von einer DNA gemäß Anspruch 4.

21. Protein (acbD-Genprodukt), kodiert von einer DNA gemäß Anspruch 5.

22. Protein (acbE-Genprodukt), kodiert von einer DNA gemäß Anspruch 6.

23. Protein (acbF-Genprodukt), kodiert von einer DNA gemäß Anspruch 7.

24. Verfahren zur Gewinnung der Proteine nach einem der Ansprüche 18 bis 23, **dadurch gekennzeichnet, daß**
(a) die Proteine in einer geeigneten Wirtszelle exprimiert und
(b) isoliert werden.

25. Verfahren gemäß Anspruch 24, **dadurch gekennzeichnet, daß** die Wirtszelle aus der Gruppe bestehend aus E. coli, Bacillus subtilis, Streptomyces-, Actinoplanes-, Ampullariella-, Streptosporangium-Stämmen, Streptomyces hygroscopicus var. limoneus, Streptomyces glaucescens, sowie Aspergillus niger, Penicillium chrysogenum und Saccharomyces cerevisiae ausgewählt wird.

26. Verfahren gemäß Anspruch 24, **dadurch gekennzeichnet, daß** die Wirtszelle aus der Gruppe bestehend aus Streptomyces glaucescens (DSM 40716) und Actinoplanes sp. ausgewählt wird.

27. Verfahren zur Herstellung von Acarbose, **dadurch gekennzeichnet, daß**
(a) eines oder mehrere Gene gemäß einem oder mehreren der Ansprüche 2 bis 7 zur Expression in einer geeigneten Wirtszelle benutzt wird und
(b) die Acarbose aus Kulturüberständen besagter Wirtszelle isoliert wird.

28. Verfahren zur Herstellung von Acarbose gemäß Anspruch 27, **dadurch gekennzeichnet, daß** Wirtszellen gemäß einem der Ansprüche 16 oder 17 gewählt werden.

29. Verfahren zur Herstellung von Acarbose, **dadurch gekennzeichnet, daß**
(a) eines oder mehrere Gene gemäß einem oder mehreren der Ansprüche 2 bis 7 in einer natürlichen Acarbose-produzierenden Wirtszelle ausgeschaltet werden und
(b) die Acarbose aus besagter Wirtszelle isoliert wird.

30. Verfahren zur Herstellung von Acarbose gemäß Anspruch 29, **dadurch gekennzeichnet**, da Wirtszellen gemäß Anspruch 17 ausgewählt werden.

31. Verfahren zur Herstellung von Acarbose, **dadurch gekennzeichnet, daß** ein Verfahren nach einem der Ansprüche 27 bis 28 mit einem Verfahren nach einem der Ansprüche 29 bis 30 kombiniert wird.

32. Verfahren zur Vervollständigung des Genclusters für die Biosynthese von Acarbose gemäß Anspruch 1, **dadurch gekennzeichnet, daß**
(a) mittels Hybridisierungsverfahren unter Verwendung von Hybridisierungssonden, die mit dem DNA-Molekül gemäß Anspruch 1 unter stringenten Bedingungen hybridisieren, angrenzende genomische DNA-Bereiche isoliert und
(b) sequenziert werden.

33. Verfahren zur Vervollständigung des Genclusters für die Biosynthese von Acarbose gemäß Anspruch 1, **dadurch gekennzeichnet, daß**
a) mittels PCR unter Verwendung von PCR-Primeren, die mit DNA-Sequenzen aus dem DNA-Molekül gemäß Anspruch 1 unter stringenten Bedingungen hybridisieren und Primern mit einer Sequenz, die Hybridisierung an Sequenzen des verwendeten Vektorsystems erlaubt, angrenzende genomische DNA-Bereiche isoliert und
b) sequenziert werden.

34. Verfahren zur Isolierung eines Genclusters für die Biosynthese von Acarbose und homologen Pseudo-Oligosacchariden aus Acarbose-produzierenden Mikroorganismen, die verschieden von Streptomyces glaucescens (DSM 40716) sind, **dadurch gekennzeichnet, daß**
a) Hybridisierungsproben, die unter stringenten Bedingungen mit dem rekombinanten DNA-Molekül gemäß Anspruch 1 hybridisieren, hergestellt werden,
b) diese Hybridisierungsproben zum genomischen oder cDNA-Screening von DNA-Bibliotheken, erhalten aus dem entsprechenden Mikroorganismus, eingesetzt werden, und
c) die gefundenen Klone isoliert und charakterisiert werden.

35. Verfahren zur Isolierung eines Genclusters für die Biosynthese von Acarbose und homologen Pseudo-Oligosacchariden aus Acarbose-produzierenden Mikroorganismen, die verschieden von Streptomyces glaucescens (DSM 40716) sind, **dadurch gekennzeichnet, daß**
a) PCR-Primer, die unter stringenten Bedingungen mit dem rekombinanten DNA-Molekül gemäß Anspruch 1 hybridisieren, hergestellt werden,
b) diese PCR-Primer zur Anreicherung von DNA-Fragmenten von genomischer DNA oder cDNA eines entsprechenden Mikroorganismus benutzt werden,
c) die angereicherten Fragmente isoliert und charakterisiert werden und
d) ggf. zu einem Verfahren nach Anspruch 34 eingesetzt werden.

36. Verfahren gemäß einem der Ansprüche 34 oder 35, **dadurch gekennzeichnet, daß** die Mikroorganismen aus der Gruppe bestehend aus Actinomycetales, Streptomyces-, Actinoplanes-, Ampullariella-, Streptosporangium-Stämmen, Streptomyces hygroscopicus var. limoneus, Streptomyces glaucescens, ausgewählt werden.

37. Verfahren gemäß Anspruch 36, **dadurch gekennzeichnet, daß** die Mikroorganismen insbesonder aus der Gruppe, bestehend aus Streptomyces glaucescens (DSM 40716) und Actinoplanes-sp. ausgewählt werden.

38. Verwendung von Streptomyces glaucescens (DSM 40716) zur Gewinnung von Acarbose.

39. Verfahren zur Veränderung der Genexpression von endogenen Acarbose-Biosynthesegenen um eine verbesserte Acarbose-Ausbeute zu erhalten, bei dem
a) Mutationen in einem oder mehreren der jeweiligen Genpromotoren eingeführt werden und
b) die Acarbose-Ausbeute des so erhaltenen Produzentenstammes mit dem Ausgangsstamm verglichen wird.

40. Verfahren gemäß Anspruch 39, **dadurch gekennzeichnet, daß** es sich bei den Mutationen um
a) Transitionen
b) Deletionen und/oder
c) Additionen
handelt.

## Claims

1. DNA molecule which comprises the genes for biosynthesizing acarbose, **characterized in that**
(a) it comprises a DNA sequence according to Table 4; or
(b) it comprises a DNA sequence which is able to hybridize, under stringent conditions, with the DNA molecule according to (a); or
(c) it comprises a DNA sequence which, because of the degeneracy of the genetic code, differs from the DNA molecules according to (a) and (b) but which permits the expression of the proteins which can be correspondingly expressed using the DNA molecules according to (a) and (b).

2. DNA molecule according to Claim 1, **characterized in that** it comprises, as the sequence mentioned under (a), the DNA sequence of nucleotides 1 to 720 (acbA gene) according to Table 4.

3. DNA molecule according to Claim 1, **characterized in that** it comprises, as the sequence mentioned under (a), the DNA sequence of nucleotides 720 to 2006 (acbB gene) according to Table 4.

4. DNA molecule according to Claim 1, **characterized in that** it comprises, as the sequence mentioned under (a), the DNA sequence of nucleotides 2268 to 3332 (acbC gene) according to Table 4.

5. DNA molecule according to Claim 1, **characterized in that** it comprises, as the sequence mentioned under (a), the DNA sequence of nucleotides 3332 to 4306 (acbD gene) according to Table 4.

6. DNA molecule according to Claim 1, **characterized in that** it comprises, as the sequence mentioned under (a), the DNA sequence of nucleotides 4380 to 5414 (acbE gene) according to Table 4.

7. DNA molecule according to Claim 1, **characterized in that** it comprises, as the sequence mentioned under (a), the DNA sequence of nucleotides 5676 to 6854 (acbF gene) according to Table 4.

8. Oligonucleotide primer for the PCR amplification of the DNA molecule according to Claim 1.

9. Vector, which comprises a DNA molecule according to one or more of Claims 1 to 8.

10. Vector according to Claim 9 for use in a process for eliminating or altering natural acarbose biosynthesis genes in an acarbose-producing microorganism.

11. Vector according to Claim 10, **characterized in that** it is selected from the group consisting of pGM160 or related vectors.

12. Vector according to Claim 9, **characterized in that** it is an expression vector and said DNA molecule is linked operatively to a promoter sequence.

13. Vector according to Claim 12, which is suitable for expression in host organisms which are selected from the group consisting of E. coli, Bacillus subtilis, Streptomyces, Actinoplanes, Ampullariella and Streptosporangium strains, Streptomyces hygroscopicus var. limoneus, Streptomyces glaucescens and also Aspergillus niger, Penicillium chrysogenum and Saccharomyces cerevisiae.

14. Vector according to Claim 12, which is suitable for expression in Streptomyces glaucescens (DSM 40716) or Actinoplanes sp.

15. Host cell which is transformed with a vector according to one of Claims 9 to 14.

16. Host cell according to Claim 15, **characterized in that** it is selected from the group consisting of E. coli, Bacillus subtilis, Streptomyces, Actinoplanes, Ampullariella or Streptosporangium strains, Streptomyces hygroscopicus var. limoneus or Streptomyces glaucescens, and also Aspergillus niger, Penicillium chrysogenum and Saccharomyces cerevisiae.

17. Host cell according to Claim 16, **characterized in that** it is selected from the group consisting of Streptomyces glaucescens (DSM 40716) and Actinoplanes sp.

18. Protein (acbA gene product), which is encoded by a DNA according to Claim 2.

19. Protein (acbB gene product), which is encoded by a DNA according to Claim 3.

20. Protein (acbC gene product), which is encoded by a DNA according to Claim 4.

21. Protein (acbD gene product), which is encoded by a DNA according to Claim 5.

22. Protein (acbE gene product), which is encoded by a DNA according to Claim 6.

23. Protein (acbF gene product), which is encoded by a DNA according to Claim 7.

24. Process for obtaining the proteins according to one of Claims 18 to 23, **characterized in that**
(a) the proteins are expressed in a suitable host cell, and
(b) are isolated.

25. Process according to Claim 24, **characterized in that** the host cell is selected from the group consisting of E. coli, Bacillus subtilis, Streptomyces, Actinoplanes, Ampullariella or Streptosporangium strains, Streptomyces hygroscopicus var. limoneus or Streptomyces glaucescens, and also Aspergillus niger, Penicillium chrysogenum and Saccharomyces cerevisiae.

26. Process according to Claim 24, **characterized in that** the host cell is selected from the group consisting of Streptomyces glaucescens (DSM 40716) and Actinoplanes sp.

27. Process for preparing acarbose, **characterized in that**
(a) one or more genes according to one or more of Claims 2 to 7 are used for expression in a suitable host cell, and
(b) the acarbose is isolated from culture supernatants of said host cell.

28. Process for preparing acarbose according to Claim 27, **characterized in that** host cells according to one of Claims 16 or 17 are selected.

29. Process for preparing acarbose, **characterized in that**
(a) one or more genes according to one or more of Claims 2 to 7 are eliminated in a natural acarbose-producing host cell, and
(b) the acarbose is isolated from said host cell.

30. Process for preparing acarbose according to Claim 29, **characterized in that** host cells according to Claim 17 are selected.

31. Process for preparing acarbose, **characterized in that** a process according to one of Claims 27 to 28 is combined with a process according to one of Claims 29 to 30.

32. Process for completing the gene cluster for biosynthesizing acarbose according to Claim 1, **characterized in that**
(a) adjoining genomic DNA regions are isolated by hybridization methods using hybridization probes which hybridize, under stringent conditions, with the DNA molecule according to Claim 5, and
(b) are sequenced.

33. Process for completing the gene cluster for biosynthesizing acarbose according to Claim 1, **characterized in that**
(a) adjoining genomic DNA regions are isolated by means of PCR using PCR primers which hybridize, under stringent conditions, with DNA sequences from the DNA molecule according to Claim 1 and primers which possess a sequence which permits hybridization to sequences of the vector system employed, and
(b) are sequenced.

34. Process for isolating a gene cluster for biosynthesizing acarbose and homologous pseudo-oligosaccharides from acarbose-producing microorganisms other than Streptomyces glaucescens (DSM 40716), **characterized in that**
a) hybridization probes which hybridize, under stringent conditions, with the recombinant DNA molecule according to Claim 1 are prepared,
b) these hybridization probes are used for the genomic or cDNA screening of DNA libraries which have been obtained from the corresponding microorganism, and
c) the clones which are found are isolated and **characterized**.

35. Process for isolating a gene cluster for biosynthesizing acarbose and homologous pseudo-oligosaccharides from acarbose-producing microorganisms other than Streptomyces glaucescens (DSM 40716), **characterized in that**,
(a) PCR primers which hybridize, under stringent conditions, with the recombinant DNA molecule according to Claim 1 are prepared,
(b) these PCR primers are used for accumulating DNA fragments of genomic DNA or cDNA from a corresponding microorganism,
(c) the accumulated fragments are isolated and **characterized**, and
(d) where appropriate, are employed for a process according to Claim 34.

36. Process according to Claim 34 or 35, **characterized in that** the microorganisms are selected from the group consisting of Actinomycetales, Streptomyces, Actinoplanes, Ampullariella and Streptosporangium strains, Streptomyces hygroscopicus var. limoneus and Streptomyces glaucescens.

37. Process according to Claim 36, **characterized in that** the microorganisms are selected, in particular, from the group consisting of Streptomyces glaucescens (DSM 40716) and Actinoplanes sp.

38. Use of Streptomyces glaucescens (DSM 40716) for obtaining acarbose.

39. Process for altering the gene expression of endogenous acarbose biosynthesis genes in order to obtain an improved acarbose yield, in which
a) mutations are introduced in one or more of the respective gene promoters, and
b) the acarbose yield of the resulting producer strain is compared with that of the starting strain.

40. Process according to Claim 39, **characterized in that** the mutations are
a) transitions
b) deletions and/or
c) additions.

## Revendications

1. Molécule d'ADN contenant les gènes pour la biosynthèse d'acarbose, **caractérisée en ce que**
(a) elle contient une séquence d'ADN selon le tableau 4; ou
(b) elle contient une séquence d'ADN qui est capable de s'hybrider dans des conditions stringentes avec la molécule d'ADN selon (a) ou
(c) elle contient une séquence d'ADN qui, en raison de la dégénérescence du code génétique, est différente des molécules d'ADN selon (a) et (b), mais qui permet l'expression des protéines exprimables conformément aux molécules d'ADN selon (a) et (b).

2. Molécule d'ADN selon la revendication 1, **caractérisée en ce qu'**en tant que la séquence mentionnée en (a) elle contient la séquence d'ADN des nucléotides 1 à 720 (gène acbA) selon le tableau 4.

3. Molécule d'ADN selon la revendication 1, **caractérisée en ce qu'**en tant que la séquence mentionnée en (a) elle contient la séquence d'ADN des nucléotides 720 à 2006 (gène acbB) selon le tableau 4.

4. Molécule d'ADN selon la revendication 1, **caractérisée en ce qu'**en tant que la séquence mentionnée en (a) elle contient la séquence d'ADN des nucléotides 2268 à 3332 (gène acbC) selon le tableau 4.

5. Molécule d'ADN selon la revendication 1, **caractérisée en ce qu'**en tant que la séquence mentionnée en (a) elle contient la séquence d'ADN des nucléotides 3332 à 4306 (gène acbD) selon le tableau 4.

6. Molécule d'ADN selon la revendication 1, **caractérisée en ce qu'**en tant que la séquence mentionnée en (a) elle contient la séquence d'ADN des nucléotides 4380 à 5414 (gène acbE) selon le tableau 4.

7. Molécule d'ADN selon la revendication 1, **caractérisée en ce qu'**en tant que la séquence mentionnée en (a) elle contient la séquence d'ADN des nucléotides 5676 à 6854 (gène acbF) selon le tableau 4.

8. Amorce oligonucléotidique pour l'amplification PCR de la molécule d'ADN selon la revendication 1.

9. Vecteur contenant une molécule d'ADN selon une ou plusieurs des revendications 1 à 8.

10. Vecteur selon la revendication 9, pour utilisation dans un procédé pour l'inactivation ou la modification de gènes naturels de la biosynthèse d'acarbose dans un micro-organisme producteur d'acarbose.

11. Vecteur selon la revendication 10, **caractérisé en ce qu'**il est choisi dans le groupe comprenant pGM160 ou des vecteurs apparentés.

12. Vecteur selon la revendication 9, **caractérisé en ce qu'**il est un vecteur d'expression et ladite molécule d'ADN est liée de façon opérationnelle à une séquence de promoteur.

13. Vecteur selon la revendication 12, approprié à l'expression dans des organismes hôtes choisis dans le groupe consistant en *E. coli*, *Bacillus subtilis,* des souches de *Streptomyces, Actinoplanes, Ampullariella, Streptosporangium, Streptomyces hygroscopicus* var. *limoneus, Streptomyces glaucescens,* ainsi *qu'Aspergillus niger, Penicillium chrysogenum* et *Saccharomyces cerevisiae.*

14. Vecteur selon la revendication 12, approprié à l'expression dans *Streptomyces glaucescens* (DSM 40716) ou *Actinoplanes sp.*

15. Cellule hôte, transformée par un vecteur selon l'une quelconque des revendications 9 à 14.

16. Cellule hôte selon la revendication 15, **caractérisée en ce qu'**elle est choisie dans le groupe consistant en *E. coli, Bacillus subtilis,* des souches de *Streptomyces, Actinoplanes, Ampullariella, Streptosporangium, Streptomyces hygroscopicus* var. *limoneus, Streptomyces glaucescens,* ainsi *qu'Aspergillus niger, Penicillium chrysogenum* et *Saccharomyces cerevisiae.*

17. Cellule hôte selon la revendication 16, **caractérisée en ce qu'**elle est choisie dans le groupe consistant en *Streptomyces glaucescens* (DSM 40716) et *Actinoplanes sp.*

18. Protéine (produit du gène acbA), codée par un ADN selon la revendication 2.

19. Protéine (produit du gène acbB), codée par un ADN selon la revendication 3.

20. Protéine (produit du gène acbC), codée par un ADN selon la revendication 4.

21. Protéine (produit du gène acbD), codée par un ADN selon la revendication 5.

22. Protéine (produit du gène acbE), codée par un ADN selon la revendication 6.

23. Protéine (produit du gène acbF), codée par un ADN selon la revendication 7.

24. Procédé pour l'obtention des protéines selon l'une quelconque des revendications 18 à 23, **caractérisé en ce que**
(a) les protéines sont exprimées dans une cellule hôte et
(b) sont isolées.

25. Procédé selon la revendication 24, **caractérisé en ce que** la cellule hôte est choisie dans le groupe consistant en *E. coli, Bacillus subtilis,* des souches de *Streptomyces, Actinoplanes, Ampullariella, Streptosporangium, Streptomyces hygroscopicus* var. *limoneus, Streptomyces glaucescens,* ainsi *qu'Aspergillus niger, Penicillium chrysogenum* et *Saccharomyces cerevisiae.*

26. Procédé selon la revendication 24, **caractérisé en ce que** la cellule hôtes est choisie dans le groupe consistant en *Streptomyces glaucescens* (DSM 40716) et *Actinoplanes sp.*

27. Procédé pour la production d'acarbose, **caractérisé en ce que**
(a) on utilise un ou plusieurs gènes selon une ou plusieurs des revendications 2 à 7, pour l'expression dans une cellule hôte appropriée et
(b) on isole l'acarbose à partir de surnageants de culture de ladite cellule hôte.

28. Procédé pour la production d'acarbose selon la revendication 27, **caractérisé en ce que** les cellules hôtes sont choisies selon la revendication 16 ou 17.

29. Procédé pour la production d'acarbose, **caractérisé en ce que**
(a) on inactive dans une cellule hôte naturelle productrice d'acarbose un ou plusieurs gènes selon une ou plusieurs des revendications 2 à 7 et
(b) on isole l'acarbose à partir de surnageants de ladite cellule hôte.

30. Procédé pour la production d'acarbose selon la revendication 29, **caractérisé en ce que** les cellules hôtes sont choisies selon la revendication 17.

31. Procédé pour la production d'acarbose, **caractérisé en ce qu'**on combine un procédé selon la revendication 27 ou 28 avec un procédé selon la revendication 29 ou 30.

32. Procédé pour complémenter le groupe de gènes pour la biosynthèse d'acarbose selon la revendication 1, **caractérisé en ce que**
(a) des régions d'ADN génomique adjacentes sont isolées par des processus d'hybridation à l'aide de sondes d'hybridation qui s'hybrident dans des conditions stringentes avec la molécule d'ADN selon la revendication 1 et
(b) sont séquencées.

33. Procédé pour complémenter le groupe de gènes pour la biosynthèse d'acarbose selon la revendication 1, **caractérisé en ce que**
(a) des régions d'ADN génomique adjacentes sont isolées par PCR à l'aide d'amorces pour PCR qui s'hybrident dans des conditions stringentes avec des séquences d'ADN provenant de la molécule d'ADN selon la revendication 1 et d'amorces ayant une séquence qui permet l'hybridation avec des séquences du système vecteur utilisé et
(b) sont séquencées.

34. Procédé pour l'isolement d'un groupe de gènes pour la biosynthèse d'acarbose et de pseudo-oligosaccharides homologues à partir de micro-organismes producteurs d'acarbose qui sont différents de *Streptomyces glaucescens* (DSM 40716), **caractérisé en ce que**
(a) on prépare des sondes d'hybridation qui s'hybrident, dans des conditions stringentes, avec la molécule d'ADN recombinant selon la revendication 1,
(b) on utilise ces sondes d'hybridation pour le criblage d'ADNc ou génomique de banques d'ADN, obtenues à partir du micro-organisme correspondant, et
(c) on isole et caractérise les clones trouvés.

35. Procédé pour l'isolement d'un groupe de gènes pour la biosynthèse d'acarbose et de pseudo-oligosaccharides homologues à partir de micro-organismes producteurs d'acarbose qui sont différents de *Streptomyces glaucescens* (DSM 40716), **caractérisé en ce que**
(a) on prépare des amorces pour PCR qui s'hybrident, dans des conditions stringentes, avec la molécule d'ADN recombinant selon la revendication 1,
(b) on utilise ces amorces pour PCR pour l'enrichissement en fragments d'ADNc ou d'ADN génomique d'un micro-organisme correspondant
(c) on isole et caractérise les fragments enrichis et
(d) éventuellement on les utilise dans un procédé selon la revendication 34.

36. Procédé selon la revendication 34 ou 35, **caractérisé en ce** les micro-organismes sont choisis dans le groupe consistant en actimomycétales des souches de *Streptomyces, Actinoplanes, Ampullariella, Streptosporangium, Streptomyces hygroscopicus* var. *limoneus, Streptomyces glaucescens.*

37. Procédé selon la revendication 36, **caractérisé en ce que** les micro-organismes sont choisis en particulier dans le groupe consistant en *Streptomyces glaucescens* (DSM 40716) et *Actinoplanes sp.*

38. Utilisation de *Streptomyces glaucescens* (DSM 40716) pour l'obtention d'acarbose.

39. Procédé pour la modification de l'expression de gènes endogènes de la biosynthèse d'acarbose pour l'obtention d'un rendement amélioré en acarbose, dans lequel
(a) on introduit des mutations dans un ou plusieurs des promoteurs de gènes respectifs et
(b) on compare le rendement en acarbose de la souche productrice ainsi obtenue avec celui de la souche initiale.

40. Procédé selon la revendication 39, **caractérisée en ce que** les mutations consistent en
a) transitions
b) délétions et/ou
c) additions.
